# EUROPEAN PATENT APPLICATION

(11) **EP 4 821 562 A2**
(43) Date of publication of application: **14.10.2026**
(21) Application number: 26185542.3
(22) Date of filing: 30.03.2018
(51) Int. Cl.: A61Q 5/12

(54) **KITS AND METHODS FOR TREATING HAIR**

(30) Priority: 31.03.2017 US 201762479916 P
(62) Document number(s) of the earlier application(s) in accordance with Art. 76 EPC: 18775021.1 / 3 630 054
(71) Applicant: L'OREAL, 75008 Paris (FR)
(72) Inventor: FACK, Geraldine, 93400 Saint-Ouen (FR); PROUST, Cecile, 93400 Saint-Ouen (FR); DEWAELE, Pierre-Alexandre, 93400 Saint-Ouen (FR); RUGHANI, Ronak, Clark, 07066 (US); APPLEBAUM, Mara, Clark, 07066 (US)
(74) Representative: Casalonga

(57) **Abstract**

The present disclosure relates to kits and methods, which are particularly useful for repairing, strengthening, and protecting hair from damage. The kits and methods employ one or more booster compositions, which can be added to a separate hair-treatment composition for application to the hair, or can be applied individually to the hair in a sequence with a separate hair-treatment composition. The booster compositions include: at least 0.5 wt.% of at least one amino acid or amino sulfonic acid, and/or a salt thereof; at least 0.5 wt.% of at least one non-polymeric mono, di, or tricarboxylic acid, and/or a salt thereof; and water. Hair treated with the kits and methods exhibit desirable cosmetic properties such as smoothness, gloss, improved combability, and enhanced strength and elasticity.

## Description

### FIELD OF THE DISCLOSURE

The instant disclosure relates to kits and methods for treating hair including, which are useful for repairing, strengthening, and protecting hair from damage. The kits and methods involve the use of booster compositions. A booster composition includes components that restructure hair fibers resulting in the hair exhibiting improved properties such as smoothness, gloss, combability, strength, and elasticity.

### BACKGROUND

Individuals desire healthy and strong hair, a healthy looking hair is in general a sign of good health and good hair-care practices. Nonetheless, nutrition, environmental influences, and chemical hair treatments can lead to hair damage that significantly weakens and dulls the hair over time. Gloss and moisture balance are deleteriously affected making the hair more difficult to comb and style. Furthermore, dry hair that has been weakened or damages is also prone to breakage and the formation of "split ends."

Nutrition plays a crucial role in the health of hair, but nutrition alone is not sufficient to compensate for the various types of physical, chemical, and environmental damage that prevent optimal hair quality. Physical hair damage is often the result of repeatedly manipulating the shape of the hair. For example, hair styles such as ponytails, buns, and braiding are quick and easy but when done too often and too tightly, can impart strain on the edges of the hair and cause a receding hair line. Hair also becomes physically damaged during detangling and styling. Excessive detangling can result in split ends and breakage.

Many chemical treatments are available for changing the appearance of hair. For example, hair may be lightened or bleached and oxidative dyes can be used to change the color of the hair. Chemical treatments for permanently straightening or curling the hair are also common. Chemical treatments are popular because their effects are long-lasting and can be drastic. Nonetheless, the biggest drawback to chemical treatments is the strain and damage they cause to the hair. This is because chemical treatments permanently change the chemical and physical structure of the hair. Another cause of chemical hair damage is heat. Repeated use of heating appliances such as flat irons and blow-dryers remove moisture from the surface of the hair cuticles, resulting in brittle, dry hair that become more vulnerable to breakage.

The environment also influences the health of hair. Regions with hard water can affect the look, feel and shine of the hair. This is because hard water leaves mineral deposits, which accumulate over time on the hair and eventually prevents moisture intake into the hair. The hair becomes dry, frizzy, and is prone to tangles. Environmental factors, such as strong sun, wind, cold air, extreme temperature variations and changes in air humidity can also damage the hair. The static and dry winter air contributes to moisture loss. Abrupt change from cold outdoor air to warm indoor air can cause the cuticle layers of the hair to lose moisture quickly into the atmosphere. Environmental effects on the hair, however, cannot be completely avoided. Thus, mechanisms to reduce or prevent damage to hair, and products that can nourish and strengthen hair are useful for combating hair damage.

### SUMMARY OF THE DISCLOSURE

The instant disclosure relates to kits and methods for treating hair, in particular, for strengthening hair and minimizing and/or compensating for damage to hair, for example, damage caused by environmental stress and/or cosmetic treatments (*e.g*., repeated washing, drying, heating, chemical processing, etc.). The kits and methods include one or more booster compositions that can be added to one or more separate hair-treatment compositions prior to application to the hair. Alternatively, the booster composition may be applied separately, for example, in a sequence with the one or more hair-treatment compositions. Hair treated with the kits and methods exhibit desirable cosmetic properties, such as improved smoothness, gloss, combability, strength, and elasticity.

Booster compositions include at least 0.5 wt.% of at least one amino acid or amino sulfonic acid, and/or a salt thereof, at least 0.5 wt.% of at least one non-polymeric mono, di, or tricarboxylic acid, and/or a salt thereof, and water. Additional components may also be included in the booster compositions. The hair-treatment compositions used in conjunction with the booster compositions relate to many types of compositions for treating the hair. For example, hair-treatment compositions include shampoos, conditioners, hair gels, hair sprays, hair rinses, hair lotions, rinse-out hair masques, etc.

In one aspect, the instant disclosure relates to kits comprising:
(A) one or more booster compositions comprising:
   (a) at least 0.5 wt.% of at least one amino acids or amino sulfonic acid, and/or a salt thereof;
   (b) at least 0.5 wt.% of at least one non-polymeric mono, di, or tricarboxylic acid, and/or a salt thereof; and
   (c) water; and
(B) one or more hair-treatment compositions;
wherein the booster composition(s) and the hair-treatment composition(s) are separately contained.

The kits and the compositions are useful in methods for treating hair, the methods comprising applying to hair one or more hair-treatment compositions and one or more booster compositions. The booster composition(s) may be mixed with one or more hair-treatment compositions prior to application to the hair, may be applied separately in a sequence, or may be layered on the hair with a hair-treatment composition. Typically, the ratio of the booster composition to the hair-treatment composition (booster composition: hair-treatment composition) is about 5:1 to about 1:5, about 1:1 to about 1:5, or about 1:1 to about 1:2.

The booster compositions and the hair-treatment compositions may include additional components, which can vary depending on the type of hair-treatment composition in which the components are incorporated. Non-limiting examples of additional component include surfactants (anionic, cationic, nonionic, amphoteric (zwitterionic), and a mixture thereof); fatty compounds; silicones; polymers including cationic polymers; thickening agents; preservatives; water-soluble solvents, etc.

The kits and methods are useful for repairing, conditioning, and protecting hair from damage, reducing/minimizing damage to hair, and/or improving the condition and quality of the hair, for example, with respect to visual /aesthetic appearance (*e.g.*, healthy look, shine, reduced split ends), feel of the hair (*e.g*., smooth feel, soft feel, conditioned feel), and manageability of the hair (*e.g*., no or less frizz, improved styleability/shapeability, ease of combing, detangling, desirable volume) . The kits and methods may be used for single treatments or for multiple treatments, *e.g*., repeatedly treating the hair with the composition(s) for one week, two weeks, one month, or longer.

### DETAILED DESCRIPTION OF THE DISCLOSURE

The instant disclosure relates to kits and methods for treating hair including, for example, repairing, strengthening, and protecting hair from damage. The term "treating hair" in the context of the instant disclosure encompasses many types of hair treatments and includes treatments for restructuring hair. The term "restructuring hair" relates to repairing hair, strengthening hair, and/or compensating for damage to hair, for example, damage due to environmental stress, cosmetic treatments (*e.g*., repeated washing, drying, heating, etc.), and chemical processing. Thus, restructuring hair involves strengthening and/or repairing hair. Restructured hair fibers exhibit desirable cosmetic properties such as, for example, improved smoothness, gloss, combability, strength, and elasticity.

In one aspect, kits of the instant disclosure include:
(A) one or more booster compositions, the one or more booster compositions comprising:
   (a) at least 0.5 wt.% of at least one amino acid or amino sulfonic acid, and/or a salt thereof;
   (b) at least 0.5 wt.% of at least one non-polymeric mono, di, or tricarboxylic acid, and/or a salt thereof; and
   (c) water; and
(B) one or more hair-treatment compositions;
wherein the booster composition(s) and the hair-treatment composition(s) are separately contained.

The kits are useful in methods for treating hair, such as methods comprising applying to hair one or more hair-treatment compositions and one or more booster compositions. The booster composition(s) may be mixed with one or more hair-treatment compositions prior to application to the hair, may be applied separately in a sequence, or may be layered with a hair-treatment composition on the hair. Typically, the ratio of booster composition to hair-treatment composition (booster composition : hair treatment composition) is about 5:1 to about 1:5, about 1:1 to about 1:5, or about 1:1 to about 1:2.

Amino acids are simple organic compounds containing both a carboxylic acid group (-COOH) and an amino group (-NH₂). Amino sulfonic acids are simple organic compounds containing both a sulfonic acid group (-SO₂OH) and an amino group (-NH₂). Accordingly, the amino acids and/or amino sulfonic acids may be selected from compounds of Formula (I) and compounds of Formula (II): 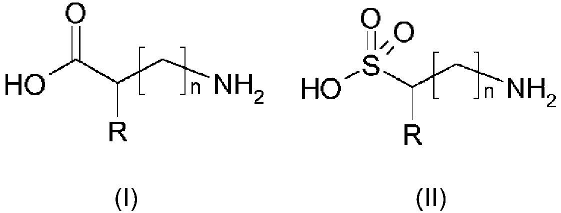
wherein R represents a hydrogen atom, a linear or branched, preferably linear, C₁-C₅ alkyl group, said alkyl group being optionally substituted with at least one group chosen from hydroxyl, -C(O)-OH, -S(O)₂-OH, -C(O)-O⁻ and M⁺, and S(O)₂-O⁻ and M⁺, where M⁺ represents a cationic counterion such as an alkali metal, alkaline earth metal, or ammonium, and n is 0 or 1. In the hair-treatment compositions containing them, the amino acids and/or amino sulfonic acids may be in their non-ionized form (I) and (II) or in their ionized or betaine form (I') and (II'): 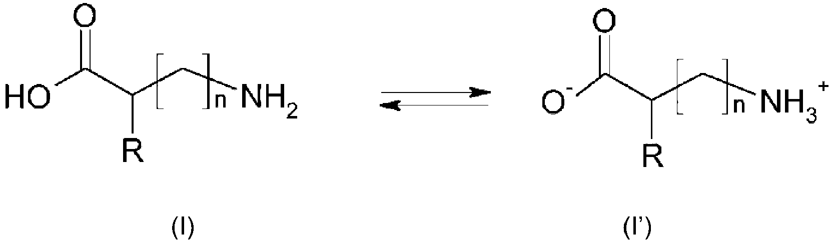 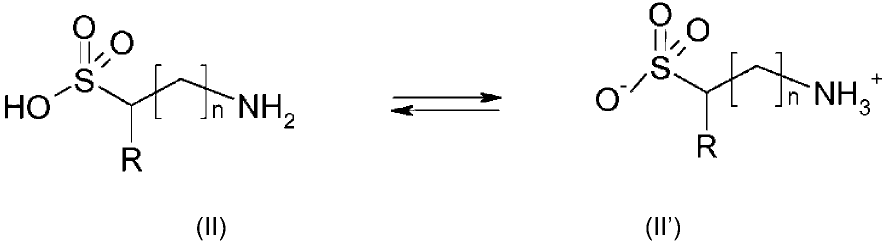
wherein "R" and "n" are as defined above. The amino acids and/or amino sulfonic acids may also be in their conjugate base form (Ib) and (IIb). 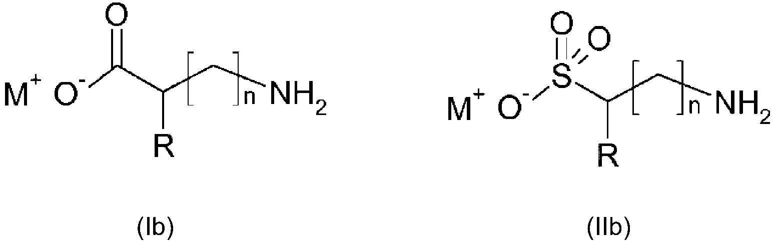
wherein "R" and "n" are as defined above.

Well-known amino acids include the twenty amino acids that form the proteins of living organisms (standard proteinogenic amino acids): alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, and valine. The amino acids of the instant disclosure, however, are not limited to the standard proteinogenic amino acids.

Non-limiting examples of amino sulfonic acids include aminomethane sulfonic acid, aminoethane sulfonic acid (taurine), aminopropane sulfonic acid, aminobutane sulfonic acid, aminohexane sulfonic acid, aminoisopropyl sulfonic acid, aminododecyl sulfonic acid, aminobenzene sulfonic acid, aminotoulene sulfonic acid, sulfanilic acid, chlorosulfanilic acid, diamino benzene sulfonic acid, amino phenol sulfonic acid, amino propyl benzene sulfonic acid, and amino hexyl benzene sulfonic acid.

In some cases, charged amino acids may be used. Non-limiting examples of charged amino acids include arginine, lysine, aspartic acid, and glutamic acid. In some cases, polar amino acids are useful. Non-limiting examples of polar amino acids include glutamine, asparagine, histidine, serine, threonine, tyrosine, cysteine, methionine, and tryptophan.

In some cases, hydrophobic amino acids may be employed. Non-limiting examples of hydrophobic amino acids include alanine, isoleucine, leucine, phenylalanine, valine, proline, and glycine.

In some instances, the booster compositions include one or more amino acids and/or amino sulfonic acids, and/or a salt thereof, selected from the group consisting of glycine, alanine, serine, beta-alanine, taurine, sodium glycinate, sodium alaninate, sodium serinate, lithium beta-alanine, sodium taurate, a salt thereof, and a mixture thereof.

In some cases, the booster compositions include one or more amino acids, and/or a salt thereof, only one or more sulfonic acids, and/or a salt thereof, or a mixture of both amino acids and sulfonic acids, and/or a salt thereof, for example, one or more amino acids and/or amino sulfonic acids selected from the group consisting of aspartic acid, cysteine, glycine, lysine, methionine, proline, tyrosine, phenylalanine, carnitine, taurine, betaine, a salt thereof, and a mixture thereof.

In some instances, the booster compositions include taurine (2-aminoethane sulfonic acid), and/or a salt thereof.

The total amount of the one or more amino acids and/or amino sulfonic acids, and/or a salt thereof, is at least 0.5 wt.%, based on the total weight of the booster composition. For example, the total amount of at least one amino acid or amino sulfonic acids, and/or a salt thereof, may be at least 0.5 wt.% to about 50 wt.%, at least 0.5 wt.% to about 40 wt.%, at least 0.5 wt.% to about 30 wt.%, at least 0.5 wt.% to about 20 wt.%, at least 0.5 wt.% to about 10 wt.%, at least 0.5 wt.% to about 5 wt.%, about 1 wt.% to about 50 wt.%, about 1 wt.% to about 40 wt.%, about 1 wt.% to about 30 wt.%, about 1 wt.% to about 20 wt.%, about 1 wt.% to about 10 wt.%, or about 1 wt.% to about 5 wt.%. In some cases, the total amount of the at least one amino acid or amino sulfonic acid is about 2 to about 10 wt.%, about 2 to about 8 wt.%, about 2 to 6 wt.%, or about 2 to 5 wt.%.

The booster compositions also typically include at least one non-polymeric mono, di, or tricarboxylic acid, and/or a salt thereof. A non-polymeric mono, di, or tricarboxylic acid is an organic compound having one (mono), two (di), or three (tri) carboxylic acid groups (-COOH). The non-polymeric mono, di, and tricarboxylic acids and salts thereof typically have a molecular weight of less than about 500 g/mol, less than about 400 g/mol, or less than about 300 g/mol.

Non-limiting examples of non-polymeric, mono, di, and tricarboxylic acids, and salt thereof, include formic acid, acetic acid, propionic acid, butyric acid, valeric acid, caproic acid, entanthic acid, caprylic acid, pelargonic acid, capric acid, undecylic acid, lauric acid, tridecylic acid, lauric acid, tridecylic acid, myristic acid, pentadecylic acid, palmitic acid, margaric acid, stearic acid, nonadecylic acid, arachidic acid, lactic acid, oxalic acid, malonic acid, malic acid, glutaric acid, citraconic acid, succinic acid, adipic acid, tartaric acid, fumaric acid, maleic acid, sebacic acid, azelaic acid, dodecanedioic acid, phthalic acid, isophthalic acid, terephthalic acid, 2,6-naphthalene dicarboxylic acid, citric acid, isocitric acid, aconitric acid, propane-1,2,3-tricarboxylic acid, benzene-1,3,5-tricarboxylic acid, a salt thereof, and a mixture thereof.

The booster compositions may include one or more mono-carboxylic acids, and/or a salt thereof. Non-limiting examples of mono-carboxylic acids, and/or salts thereof, include formic acid, acetic acid, propionic acid, butyric acid, valeric acid, caproic acid, entanthic acid, caprylic acid, pelargonic acid, capric acid, undecylic acid, lauric acid, tridecylic acid, lauric acid, tridecylic acid, myristic acid, pentadecylic acid, palmitic acid, margaric acid, stearic acid, nonadecylic acid, arachidic acid, lactic acid, a salt thereof, and a mixture thereof.

The booster compositions may include one or more di-carboxylic acids, and/or a salt thereof. Non-limiting examples of di-carboxylic acids, and/or salt thereof, include oxalic acid, malonic acid, malic acid, glutaric acid, citraconic acid, succinic acid, adipic acid, tartaric acid, fumaric acid, maleic acid, sebacic acid, azelaic acid, dodecanedioic acid, phthalic acid, isophthalic acid, terephthalic acid, 2,6-naphthalene dicarboxylic acid, and a mixture thereof. In some instances, the hair-treatment compositions include maleic acid, malonic acid, malic acid, oxalic acid, a salt thereof, and a mixture thereof.

The booster compositions may include one or more tricarboxylic acids, and/or a salt thereof. Non-limiting examples of tricarboxylic acids, and/or salt thereof, include citric acid, isocitric acid, aconitric acid, propane-1,2,3-tricarboxylic acid, benzene-1,3,5-tricarboxylic acid, a salt thereof, and a mixture thereof. In some instances, the hair-treatment compositions include at least citric acid, and/or a salt thereof.

The total amount of the at least one non-polymeric mono, di, or tricarboxylic acid, and/or a salt thereof, is at least 0.5 wt.%, based on the total weight of the hair-treatment booster composition. In some cases, the total amount of the at least one non-polymeric mono, di, or tricarboxylic acid, and/or a salt thereof, is at least 0.6, 0.7, 0.8, 0.9, or 1 wt.% up to about 15, 20, 25, or 30 wt.%. Furthermore, the total amount of the at least one non-polymeric mono, di, or tricarboxylic acid, and/or a salt thereof, may be at least 0.5 wt.% to about 50 wt.%, at least 0.5 wt.% to about 40 wt.%, at least 0.5 wt.% to about 30 wt.%, at least 0.5 wt.% to about 20 wt.%, at least 0.5 wt.% to about 10 wt.%, at least 0.5 wt.% to about 5 wt.%, at least 0.8 wt.% to about 50 wt.%, at least 0.8 wt.% to about 40 wt.%, at least 0.8 wt.% to about 30 wt.%, about 0.8 to about 20 wt.%, about 0.8 to about 10 wt.%, about 0.8 wt.% to about 5 wt.%, about 1 wt.% to about 50 wt.%, about wt.% to about 40 wt.%, about 1 wt.% to about 30 wt.%, about 1 wt.% to about 20 wt.%, about 1 wt.% to about 10 wt.%, about 1 wt.% to about 5 wt.%, about 2 wt.% to about 50 wt.%, about 2 wt.% to about 40 wt.%, about 2 wt.% to about 30 wt.%, about 2 wt.% to about 20 wt.%, about 2 wt.% to about 10 wt.%, or about 2 wt.% to about 5 wt.%.

The total amount of water in the booster compositions may vary but is typically about 50 to about 99 wt.%, based on the total weight of the booster composition, including all ranges and subranges therebetween. The total amount of water may be about 60 to about 99 wt.%, about 70 to about 99 wt.%, about 75 to about 99 wt.%, about 80 to about 99 wt.%, about 50 to about 95 wt.%, about 60 to about 95 wt.%, about 70 to about 95 wt.%, about 75 to about 95 wt.%, or about 80 to about 95 wt.%.

The booster compositions may optionally include one or more surfactants. The various surfactants that may be used with the booster composition(s) are the same as those that may be included in the hair-treatment compositions, discussed in more detail later.

A "hair-treatment composition" is a composition that is applied to the hair to achieve a particular cosmetic effect. Non-limiting examples of hair-treatment compositions include shampoos, conditioners, hair gels, hair sprays, hair rinses, hair lotions, rinse-out hair masques, etc. The booster compositions of the instant disclosure are also considered a type of hair-treatment composition. Hair-treatment compositions are often rinsed from the hair after a period of time, *e.g*., after the hair-treatment composition has had sufficient time to impart the desired cosmetic effect to the hair and may therefore be referred to in these instances as a "rinse-out composition" (or a "rinse-out product"). A "rinse-out" composition is a composition that is applied to the hair to provide a desired treatment (*e.g*., cleansing, conditioning, restructuring, shining, smoothing, shaping, etc.) but is not intended to remain on the hair indefinitely. Typically, a rinse-out composition is rinsed from the hair before styling the hair. Shampoos and conditioners are well-known examples of rinse-out compositions but the rinse-out compositions of the instant disclosure are not limited to shampoos and conditioners. For instance, rinse-out compositions may additionally include, for example, gels, sprays, rinses, lotions, rinse-out hair masques, etc.

The hair-treatment compositions according to the instant disclosure except for the booster compositions, may include at least one amino acid or amino sulfonic acid, and/or a salt thereof, and may include at least one non-polymeric mono, di, or tricarboxylic acid, and/or a salt thereof, but these components are not required.

The hair-treatment compositions may include one or more surfactants. Cationic, anionic, amphoteric/zwitterionic, and nonionic surfactants, and combination thereof, may be used. In some cases anionic surfactants are included in the hair-treatment compositions, especially if the hair-treatment composition is a shampoo. One or more amphoteric surfactants may be used together with one or more anionic surfactants. In some cases, cationic surfactants are useful, especially if the hair-treatment composition is a conditioner.

The total amount of the one or more surfactants included in the hair-treatment compositions can vary depending on the type of hair-treatment composition in which they are contained. Nonetheless, in some cases, the total amount of total surfactant(s) is about 0.1 to about 40 wt.%, based on the total weight of the hair-treatment composition, including all ranges and subranges therebetween. In some cases, the total amount of the one or more surfactants is about 0.1 to about 35 wt.%, about 0.1 to about 30 wt.%, about 0.1 to about 25 wt.%, about 0.1 to about 20 wt.%, about 0.1 to about 15 wt.%, about 0.1 to about 10 wt.%, about 0.1 to about 5 wt.%, about 0.5 to about 40 wt.%, about 0.5 to about 35 wt.%, about 0.5 to about 30 wt.%, about 0.5 to about 25 wt.%, about 0.5 to about 20 wt.%, about 0.5 to about 15 wt.%, about 0.5 to about 10 wt.%, about 0.5 to about 5 wt.%, about 1 wt.% to about 40 wt.%, about 1 wt.% to about 30 wt.%, about 1 wt.% to about 20 wt.%, about 1 wt.% to about 15 wt.%, about 1 wt.% to about 10 wt.%, or about 1 wt.% to about 5 wt.%.

The hair-treatment compositions may include one or more anionic surfactants. Anionic surfactants are particularly useful in shampoos. Non-limiting examples of anionic surfactants include alkyl sulfates, alkyl ether sulfates, acyl isethionates, acyl glycinates, acyl taurates, acyl amino acids, acyl sarcosinates, sulfosuccinates, sulfonates, and a mixture thereof, wherein the alkyl and acyl groups of all these compounds comprise from 6 to 24 carbon atoms. In some cases, anionic sulfate surfactants may be excluded from the one or more anionic surfactants. In such cases, the one or more anionic surfactants may be selected from the group consisting of acyl isethionates, acyl glycinates, acyl taurates, acyl amino acids, acyl sarcosinates, sulfosuccinates, sulfonates, and a mixture thereof, wherein the alkyl and acyl groups of all these compounds comprise from 6 to 24 carbon atoms. A more exhaustive list of anionic surfactants that may be included in the hair-treatment compositions is provided later, under the heading "Anionic Surfactants."

The total amount of the one or more anionic surfactants may be about 1 to about 40 wt.%, based on the total weight of the hair-treatment composition, including all ranges and subranges therebetween. Furthermore, the total amount of the one or more anionic surfactants may be about 1 to about 35 wt.%, about 1 to about 30 wt.%, about 5 wt.% to about 40 wt.%, about 5 wt.% to about 25 wt.%, about 5 wt.% to about 30 wt.%, about 10 wt.% to about 40 wt.%, about 10 wt.% to about 35 wt.%, or about 15 wt.% to about 40 wt.%.

One or more amphoteric surfactants may be included in the hair-treatment compositions. Non-limiting examples of amphoteric surfactants include betaines, sultaines, amphoacetates, amphoproprionates, and a mixture thereof. In some cases, the hair-treatment compositions include one or more betaines, for example, alkyl betaines, alkyl amidopropyl betaines, alkyl sulphobetaines (sultaines), and a mixture thereof. In some cases, one or more amphoteric surfactants may be used together with one or more anionic surfactants. A more exhaustive list of amphoteric surfactants that may be included in the hair-treatment compositions is provided later, under the heading "Amphoteric Surfactants."

When one or more amphoteric surfactants are included in the hair-treatment compositions the total amount of the one or more amphoteric surfactants is typically about 0.1 to about 20 wt.%, based on the total weight of the hair-treatment composition, including all ranges and subranges therebetween. Additionally, the total amount of the one or more amphoteric surfactants may be about 0.1 to about 15 wt.%, about 0.1 to about 10 wt.%, about 0.1 to about 5 wt.%, about 0.1 to about 3 wt.%, about 0.5 to about 15 wt.%, about 0.5 to about 10 wt.%, about 0.5 to about 5 wt.%, about 0.5 to about 3 wt.%, about 1 wt.% to about 15 wt.%, about 1 wt.% to about 10 wt.%, or about 1 wt.% to about 5 wt.%.

One or more cationic surfactants may be included in hair-treatment compositions. Non-limiting examples of cationic surfactants include cetrimonium chloride, stearimonium chloride, behentrimonium chloride, behentrimonium methosulfate, behenamidopropyltrimonium methosulfate, stearamidopropyltrimonium chloride, arachidtrimonium chloride, distearyldimonium chloride, dicetyldimonium chloride, tricetylmonium chloride, oleamidopropyl dimethylamine, linoleamidopropyl dimethylamine, isostearamidopropyl dimethylamine, oleyl hydroxyethyl imidazoline, stearamidopropyldimethylamine, behenamidopropyldimethylamine, behenamidopropyldiethylamine, behenamidoethyldiethyl-amine, behenamidoethyldimethylamine, arachidamidopropyldimethylamine, arachidamido-propyidiethylamine, arachidamidoethyidiethylamine, arachidamidoethyidimethylamine, and a mixture thereof. A more exhaustive list of cationic surfactants that may be included in the hair-treatment compositions is provided later, under the heading "Cationic Surfactants."

When one or more cationic surfactants is included in the hair-treatment compositions, the total amount of the one or more cationic surfactants is typically about 0.1 to about 20 wt.%, based on the total weight of the hair-treatment composition, including all ranges and subranges therebetween. Additionally, the total amount of the one or more cationic surfactants may be about 0.1 to about 15 wt.%, about 0.1 to about 10 wt.%, about 0.1 to about 5 wt.%, about 0.1 to about 3 wt.%, about 0.5 to about 15 wt.%, about 0.5 to about 10 wt.%, about 0.5 to about 5 wt.%, about 0.5 to about 3 wt.%, about 1 wt.% to about 15 wt.%, about 1 wt.% to about 10 wt.%, or about 1 wt.% to about 5 wt.%.

The total amount of water in the hair-treatment compositions may vary depending on the type of composition and the desired consistency, viscosity, etc. In some cases, the total amount of water is about 50 to 95 wt.%, based on the total weight of the hair-treatment composition, including all ranges and subranges therebetween. The total amount of water may be about 50 to about 90 wt.%, about 50 to about 85 wt.%, about 60 to 95 wt.%, about 60 to 90 wt.%, about 60 to about 85 wt.%, greater than 60 to about 95 wt.%, greater than 60 to about 90 wt.%, greater than 60 to about 85 wt.%, about 65 to about 95 wt.%, about 65 to about 90 wt.%, about 65 to about 85 wt.%, about 70 to about 95 wt.%, or about 70 to about 90 wt.%. In some instances, especially when the hair-treatment composition is a shampoo, the total amount of water is greater than 60 wt.%. For example, the total amount of water may be at least 61, 62, 63, 64, 65, 66, 67, 68, 69, or 70 wt.% up to about 90 or 95 wt.%. Likewise, the total amount of water may be at least 61, 62, 63, 64, 65, 66, 67, 68, 69, or 70 wt.% to about 95 wt.%, 61, 62, 63, 64, 65, 66, 67, 68, 69, or 70 wt.% to about 90 wt.%, or about 70 wt.% to about 90 wt.%.

In some cases, the kits of the instant disclosure comprise: (A) a booster composition; and either or both of (B) a shampoo and (C) a conditioner. For example, a kit may include: (A) a booster composition and (B) a shampoo (but not (C) a conditioner). Alternatively, a kit may include: (A) a booster compositions and (C) a conditioner (but not (B) a shampoo). Nonetheless, a kit may include all three compositions: (A) a booster composition, (B) a shampoo composition, and (C) a conditioning composition. In one aspect, the kits of the instant disclosure include:
(A) a booster composition comprising:
   (a) at least 0.5 to about 25 wt.% of at least one amino acid or amino sulfonic acid, and/or a salt thereof; and
   (b) at least 0.5 wt.% to about 25 wt.% of at least one non-polymeric mono, di, or tri-carboxylic acid, and/or a salt thereof;
   (c) optionally, about 0.1 to about 10 wt.% of one or more surfactants selected from the group consisting of nonionic surfactants, cationic surfactants, and a mixture thereof; and
   (d) water;
(B) a shampoo comprising:
   (a) about 1 to about 25 wt.% of one or more surfactants selected from the group consisting of anionic surfactants, amphoteric surfactants, cationic surfactants, and a mixture thereof; and
   (b) water; and/or
(C) a conditioner comprising;
   (a) about 1 to about 15 wt.% of one or more cationic surfactants; and
   (b) water;
   wherein the booster composition, the shampoo, and the conditioner are separately contained.

The booster composition may be mixed with the shampoo, the conditioner, or mixed with the shampoo and the conditioner prior to using the shampoo and/or conditioner. Alternatively, the booster composition may be applied separately to the hair in a sequence. For example, the booster composition may be applied to the hair before the shampoo (as a pre-treatment), after the shampoo but before the conditioner (as an intermediate treatment), or after conditioning (a post-treatment). The booster composition may also be applied at the same time as the shampoo and/or the conditioner, *i.e*., the booster compositions is not necessarily mixed with the shampoo and/or conditioner prior to application to the hair but is applied to the hair while shampoo and/or conditioner is also on the hair. This may involve layering the booster composition with the shampoo and/or conditioner on the hair.

The booster composition or another hair-treatment composition comprising the booster composition (*e.g*., a conditioner or shampoo that has been pre-mixed with the booster composition) may be applied to the hair and allowed to remain on the hair for a period of time before being rinsed from the hair. For example, such compositions may be applied to the hair and allowed to remain on the hair for about 5 seconds to about 30 minutes, about 5 second to about 20 minutes, about 5 seconds to about 10 minutes, about 5 seconds to about 5 minutes, about 5 seconds to about 1 minute, about 15 seconds to about 30 minutes, about 15 seconds to about 20 minutes, about 15 seconds to about 10 minutes, about 15 seconds to about 5 minutes, about 15 seconds to about 1 minute, about 30 seconds to about 30 minutes, about 30 seconds to about 20 minutes, about 30 seconds to about 10 minutes, about 30 seconds to about 5 minutes, about 30 seconds to about 1 minute, about 1 minute to about 30 minutes, about 1 minute to about 20 minutes, about 1 minute to about 10 minutes, or about 1 minute to about 5 minutes.

In one aspect, methods of treating hair according to the instant disclosure include hair cleansing and conditioning routines carried out in conjunction with the use of a booster composition. Such methods include, for example,
(i) cleansing the hair with a shampoo;
(ii) conditioning the hair with a conditioner; and
(iii) applying a booster composition to the hair.

The booster composition may be applied to the hair individually. Alternatively, the booster composition may be mixed with the shampoo prior to cleansing the hair with the shampoo, the booster composition may be mixed with the conditioner prior to conditioning the hair, or the booster composition may be mixed with both the shampoo and the conditioner prior to cleansing and conditioning the hair. In some instances, when mixing the booster with the shampoo and/or conditioner, the ratio of booster to shampoo (or to conditioner) is about 5:1 to about 1:5, about 2:1 to about 1:5; about 2:1 to about 1:3, about 1:1 to about 1:5, about 1:1 to about 1:3, or about 1:1 to about 1:2 (booster composition : shampoo (or conditioner)).

Methods for treating hair according to the instant disclosure also include:
A. mixing a booster composition with a shampoo and/or a conditioner prior to application of the shampoo and/or conditioner to the hair, the booster compositions comprising:
   (a) at least 0.5 wt.%, at least 0.7 wt.%, or about 1 to about 6 wt.% of at least one amino acid or amino sulfonic acid, and/or a salt thereof, such as taurine, and/or a salt thereof; and
   (b) at least 0.5 wt.%, at least 0.7 %, or about 1 to about 6 wt.% of at least one non-polymeric mono, di, or tricarboxylic acid, and/or a salt thereof, for example one or more tricarboxylic acids, and/or a salt thereof, such as citric acid, and/or a salt thereof; and
   (c) water, for example, about 50 to about 95 wt.%, about 60 to about 95 wt.%, or about 70 to about 95 wt.% water; and
B. cleansing the hair with the mixed shampoo and/or conditioning the hair with the mixed conditioner,
   the shampoo comprising:
      (a) about 1 to about 25 wt.%, about 1 to about 20 wt.%, or about 1 to about 15 wt.% of one or more surfactants selected from the group consisting of anionic surfactants, amphoteric surfactants, cationic surfactants, and a mixture thereof; and
      (b) water, for example, about 50 to about 95 wt.%, about 60 to about 95 wt.%, or about 70 to about 95 wt.% water;
   the conditioner comprising:
      (a) about 1 to about 15 wt.%, about 1 to about 10 wt.%, or about 1 to about 8 wt.% of one or more cationic surfactants; and
      (b) water, for example, about 50 to about 95 wt.%, about 60 to about 95 wt.%, or about 70 to about 95 wt.% water.

One or more fatty compounds can be included in the hair-treatment compositions. Non-limiting examples of fatty compounds include oils, mineral oil, alkanes, fatty alcohols, fatty acids, fatty alcohol derivatives, fatty acid derivatives (such as alkoxylated fatty acids or polyethylene glycol esters of fatty acids or propylene glycol esters of fatty acids or butylene glycol esters of fatty acids or esters of neopentyl glycol and fatty acids or polyglycerol/glycerol esters of fatty acids or glycol diesters or diesters of ethylene glycol and fatty acids or esters of fatty acids and fatty alcohols, esters of short chain alcohols and fatty acids), esters of fatty alcohols, hydroxy-substituted fatty acids, waxes, triglyceride compounds, lanolin, ceramide, and a mixture thereof. For instance, one or more fatty compounds may be selected from the group consisting of glycol distearate, PEG-55 propylene glycol oleate, cetearyl alcohol, soybean oil, cetyl esters, isopropyl myristate, cetearyl alcohol, orbigynya oleifera seed oil, propylene glycol dicaprylate/dicaprate, mineral oil, undecane, tridecane, 2-oleamido-1,3-octadecanediol (ceramide), and a mixture thereof.

Non-limiting examples of the fatty alcohols, fatty acids, fatty alcohol derivatives, and fatty acid derivatives are found in International Cosmetic Ingredient Dictionary, Sixteenth Edition, 2016, which is incorporated by reference herein in its entirety.

Fatty alcohols useful herein include those having from about 10 to about 30 carbon atoms, from about 12 to about 22 carbon atoms, and from about 16 to about 22 carbon atoms. These fatty alcohols can be straight or branched chain alcohols and can be saturated or unsaturated. Non-limiting examples of fatty alcohols include decyl alcohol, undecyl alcohol, dodecyl, myristyl, cetyl alcohol, stearyl alcohol, isostearyl alcohol, isocetyl alcohol, behenyl alcohol, linalool, oleyl alcohol, cholesterol, cis4-t-butylcyclohexanol, myricyl alcohol and a mixture thereof. In some cases, the fatty alcohols are those selected from the group consisting of cetyl alcohol, stearyl alcohol, isostearyl alcohol, oleyl alcohol, and a mixture thereof.

Fatty acids useful herein include those having from about 10 to about 30 carbon atoms, from about 12 to about 22 carbon atoms, and from about 16 to about 22 carbon atoms. These fatty acids can be straight or branched chain acids and can be saturated or unsaturated. Also included are diacids, triacids, and other multiple acids which meet the carbon number requirement herein. Also included herein are salts of these fatty acids. Nonlimiting examples of fatty acids include lauric acid, palmitic acid, stearic acid, behenic acid, arichidonic acid, oleic acid, isostearic acid, sebacic acid, and a mixture thereof. In some cases, the fatty acids are selected from the group consisting of palmitic acid, stearic acid, and a mixture thereof.

Fatty alcohol derivatives include alkyl ethers of fatty alcohols, alkoxylated fatty alcohols, alkyl ethers of alkoxylated fatty alcohols, esters of fatty alcohols and a mixture thereof. Nonlimiting examples of fatty alcohol derivatives include materials such as methyl stearyl ether; 2-ethylhexyl dodecyl ether; stearyl acetate; cetyl propionate; the ceteth series of compounds such as ceteth-1 through ceteth-45, which are ethylene glycol ethers of cetyl alcochol, wherein the numeric designation indicates the number of ethylene glycol moieties present; the steareth series of compounds such as steareth-1 through 10, which are ethylene glycol ethers of steareth alcohol, wherein the numeric designation indicates the number of ethylene glycol moieties present; ceteareth 1 through ceteareth-10, which are the ethylene glycol ethers of ceteareth alcohol, i.e. a mixture of fatty alcohols containing predominantly cetyl and stearyl alcohol, wherein the numeric designation indicates the number of ethylene glycol moieties present; C1-C30 alkyl ethers of the ceteth, steareth, and ceteareth compounds just described; polyoxyethylene ethers of branched alcohols such as octyldodecyl alcohol, dodecylpentadecyl alcohol, hexyldecyl alcohol, and isostearyl alcohol; polyoxyethylene ethers of behenyl alcohol; PPG ethers such as PPG-9-steareth-3, PPG-11 stearyl ether, PPG8-ceteth-1, and PPG-10 cetyl ether; and mixtures of all of the foregoing compounds.

Non-limiting polyglycerol esters of fatty acids include those of the following formula: 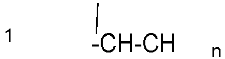 wherein the average value of n is about 3 and R¹, R² and R³ each may independently be a fatty acid moiety or hydrogen, provided that at least one of R¹, R², and R³ is a fatty acid moiety. For instance, R¹, R² and R³ may be saturated or unsaturated, straight or branched, and have a length of C₁-C₄₀, C₁-C₃₀, C₁-C₂₅, or C₁-C₂₀, C₁-C_{16, or} C₁-C₁₀. For example, glyceryl monomyristate, glyceryl monopalmitate, glyceryl monostearate, glyceryl isostearate, glyceryl monooleate, glyceryl ester of mono(olive oil fatty acid), glyceryl dioleate and glyceryl distearate. Additionally, non-limiting examples of nonionic polyglycerol esters of fatty acids include polyglyceryl-4 caprylate/caprate, polyglyceryl-10 caprylate/caprate, polyglyceryl-4 caprate, polyglyceryl-10 caprate, polyglyceryl-4 laurate, polyglyceryl-5 laurate, polyglyceryl-6 laurate, polyglyceryl-10 laurate, polyglyceryl-10 cocoate, polyglyceryl-10 myristate, polyglyceryl-10 oleate, polyglyceryl-10 stearate, and a mixture thereof.

The fatty acid derivatives are defined herein to include fatty acid esters of the fatty alcohols as defined above, fatty acid esters of the fatty alcohol derivatives as defined above when such fatty alcohol derivatives have an esterifiable hydroxyl group, fatty acid esters of alcohols other than the fatty alcohols and the fatty alcohol derivatives described above, hydroxy-substitued fatty acids, and a mixture thereof. Nonlimiting examples of fatty acid derivatives inlcude ricinoleic acid, glycerol monostearate, 12-hydroxy stearic acid, ethyl stearate, cetyl stearate, cetyl palmitate, polyoxyethylene cetyl ether stearate, polyoxyethylene stearyl ether stearate, polyoxyethylene lauryl ether stearate, ethyleneglycol monostearate, polyoxyethylene monostearate, polyoxyethylene distearate, propyleneglycol monostearate, propyleneglycol distearate, trimethylolpropane distearate, sorbitan stearate, polyglyceryl stearate, dimethyl sebacate, PEG-15 cocoate, PPG-15 stearate, glyceryl monostearate, glyceryl distearate, glyceryl tristearate, PEG-8 laurate, PPG-2 isostearate, PPG-9 laurate, and a mixture thereof. Preferred for use herein are glycerol monostearate, 12-hydroxy stearic acid, and a mixture thereof.

When one or more fatty compounds are included in the hair-treatment compositions, the total amount of the one or more fatty compounds may be about 0.1 to about 40 wt.%, based on the total weight of the hair-treatment composition, including all ranges and subranges therebetween. In some cases, the total amount of the one or more fatty compounds may be about 0.1 to about 30 wt.%, about 0.1 to about 20 wt.%, about 0.1 to about 20 wt.%, about 0.1 to about 10 wt.%, about 1 wt.% to about 40 wt.%, about 1 wt.% to about 30 wt.%, about 1 wt.% to about 20 wt.%, or about 1 wt.% to about 10 wt.%.

The hair-treatment compositions may also include one or more silicones. Non-limiting examples of silicones include polyorganosiloxanes, polyalkylsiloxanes, polyarylsiloxanes, polyalkarylsiloxanes, polyestersiloxanes, and mixtures thereof. In particular, suitable examples of silicones include dimethicone***,*** cyclomethicone, amodimethicone, trimethyl silyl amodimethicone***,*** phenyl trimethicone, trimethyl siloxy silicate, and mixtures thereof. A more exhaustive list of silicones that may be included in the hair-treatment compositions is provided later, under the heading "Silicones."

The total amount of the one or more silicones can vary but is typically about 0.1 to about 40 wt.%, based on the total weight of the composition, including all ranges and subranges therebetween. In some cases, the total amount of the one or more silicones is about 0.1 to about 30 wt.%, about 0.1 to about 20 wt.%, about 0.1 to about 10 wt.%, about 0.1 to about 5 wt.%, about 1 to about 30 wt.%, about 1 to about 20 wt.%, about 1 to about 15 wt.%, about 1 to about 10 wt.%, or about 1 to about 5 wt.%.

The hair-treatment compositions may also include one or more alkylamines and/or alkanolamines. Non-limiting exaamples of alkylamines and alkanolamines include those of the following formula:

NR₃R₄R₅

wherein R₃, R₄ and R₅ are independently H, C₁-C₄₀ alkyl, C₁-C₄₀ monohydroxyalkyl or C₂-C₄₀ polyhydroxyalkyl, provided that at least one of R₃, R₄ and R₅ is an alkyl or mono or polyhydroxyalkyl. Further R₃, R₄ and R₅ may also independently be H, C₁-C₂₀ alkyl, C₁-C₂₀ monohydroxyalkyl or C₂-C₂₀ polyhydroxyalkyl, provided that at least one of R₃, R₄ and R₅ is an alkyl or mono or polyhydroxyalkyl. Also, R₃, R₄ and R₅ may also independently be H, C₁-C₁₀ alkyl, C₁-C₁₀ monohydroxyalkyl or C₂-C₁₀ polyhydroxyalkyl, provided that at least one of R₃, R₄ and R₅ is an alkyl or mono or polyhydroxyalkyl.

In some cases, the hair-treatment compositions include one or more alkanolamines. Non-limiting examples include monoethanol amine, diethanolamine, triethanolamine, monoisopropanolamine, diisopropanolamine, N-dimethylamino-ethanolamine, 2-amino-2-methyl-1-propanol, triisopropanolamine, 2-amino-2-methyl-1,3-propanediol, 3-amino-1,2-propanediol, 3-dimethylamino-1,2-propanediol, tris(hydroxymethylamino) methane, and a mixture thereof.

The total amount of the one or more alkylamines and/or alkanolamines can vary but is typically about 0.1 to about 20 wt.%, based on the total weight of the composition, including all ranges and subranges therebetween. The total amount of the one or more alkylamines and/or alkanolamines may be about 0.1 to about 15 wt.%, about 0.1 to about 10 wt.%, or about 0.1 to about 5 wt.%.

In some instances, the hair-treatment compositions include one or more cationic polymers. Non-limiting examples of cationic polymers include poly(methacryloyloxyethyl trimethylammonium chloride), polyquaternium-37, quaternized cellulose derivatives, polyquaternium-4, polyquaternium-10, cationic alkyl polyglycosides, cationized honey, cationic guar derivatives, polymeric dimethyl diallyl ammonium salts and copolymers thereof with esters and amides of acrylic acid and methacrylic acid, copolymers of vinyl pyrrolidone with quaternized derivatives of dialkylaminoalkyl acrylate and methacrylate, vinyl pyrrolidone-vinyl imidazolium methochloride copolymers, quaternized polyvinyl alcohol, polyquaternium-2, polyquaternium-7, polyquaternium-17, polyquaternium-18, polyquaternium-24, polyquaternium-27, and a mixture thereof. In some instances, the one or more cationic polymers may be selected from the group consisting of polyquaternium-4, polyquaternium-10, cationic guar derivatives, and a mixture thereof.

The cationic polymers can be a monoalkyl quaternary amine, such as stearyltrimonium chloride, soyatrimonium chloride or coco-ethyldimonium ethosulfate. Other suitable cationic polymers include, but are not limited to, behentrimonium chloride, dialkyl quaternary amines, such as dicetyldimonium chloride, dicocodimethyl ammonium chloride or distearyldimethyl ammonium chloride; and polyquaternium compounds, such as Polyquaternium-6, Polyquaternium-22 or Polyquaternium-5.

For example, cationic polymers may be chosen from polyquaterium-10 (also called quaternized polyhydroxyethyl cellulose), cetrimonium chloride (also called cetyl trimethyl ammonium chloride, CTAC), behentrimonium chloride (also known as docosyl trimethyl ammonium chloride), behentrimonium methosulfate, steartrimonium chloride, stearalkonium chloride, dicetyldimonium chloride, hydroxypropyltrimonium chloride, cocotrimonium methosulfate, olealkonium chloride, steartrimonium chloride, babassuamidopropalkonium chloride, brassicamidopropyl dimethylamine, Quaternium-91, Salcare/PQ-37, Quaternium-22, Quaternium-87, Polyquaternium-4, Polyquaternium-6, Polyquaternium-11, Polyquaternium-44, Polyquaternium-67, amodimethicone, lauryl betaine, Polyacrylate-1 Crosspolymer, steardimonium hydroxypropyl hydrolyzed wheat protein, behenamidopropyl PG-dimonium chloride, lauryldimonium hydroxypropyl hydrolyzed soy protein, aminopropyl dimethicone, Quaterium-8, and dilinoleamidopropyl dimethylamine dimethicone PEG-7 phosphate.

In some instances, the cationic polymers are cationic conditioning polymers. Examples of cationic conditioning polymers that can be used include, without limitation, cationic cellulose, cationic proteins, and cationic polymers. The cationic polymers can have a vinyl group backbone of amino and/or quaternary ammonium monomers. Cationic amino and quaternary ammonium monomers include, without limitation, dialkylamino alkylmethacrylate, monoalkylaminoalkyl acrylate, monoalkylaminoalkyl methacrylate, trialkyl methacryoloxyalkyl ammonium salt, trialkyl acryloxyalkyl ammonium salts, diallyl quaternary ammonium salts, vinyl compounds substituted with dialkyl aminoalkyl acrylate, and vinyl quaternary ammonium monomers having cyclic cationic nitrogen containing rings such as pyridinium, imidazolium, or quaternized pyrrolidine. Other examples of cationic conditioning polymers that can be used include, without limitation, hydroxypropyltrimonium honey, cocodimonium silk amino acids, cocodimonium hydroxypropyl hydrolyzed wheat or silk protein, polyquaternium-5, polyquaternium-11, polyquaternium-2, polyquaternium-4, polyquaternium-6, polyquaternium-7, polyquaternium-14, polyquaternium-16, polyquaternium-22, polyquaternium-10, and guar hydroxypropyltrimonium chloride.

In some cases quaternized polymeric cationic polymers are particularly useful. Particularly preferred are quaternary nitrogen polymers prepared by the polymerization of a dialkyldiallylammonium salt or copolymer thereof in which the alkyl group contains 1 to about 18 carbon atoms, and more preferably where the alkyl group is methyl or ethyl. Details concerning the preparation of these polymers can be found in U.S. Pat. Nos. 3,288,770, 3,412,019 and 4,772,462, incorporated herein by reference. For example, cationic homopolymers and copolymers of polydiallyldimethylammonium chloride are available in aqueous compositions sold under the trademark MERQUAT by the Calgon Corporation, subsidiary of Merck & Co., Pittsburgh, Pa. The homopolymer, which is named Polyquaternium-6 is sold under the trademark MERQUAT-100, and is described as having a weight average molecular weight of approximately 100,000. A copolymer reaction product of dimethyldiallylammonium chloride with acrylamide monomers is named Polyquaternium-7 is described as having a weight average molecular weight of approximately 500,000 and is sold under the trademark MERQUAT-550. Another copolymer reaction product of dimethyldiallylammonium chloride with acrylic acids having a weight average molecular weight from about 50,000 to about 10,000,000 has the name Polyquaternium-22 and is sold under the trademark MERQUAT-280. Polyquaternium-6 is particularly preferred.

Other polymeric conditioners include cationic copolymers of methylvinylimidazolium chloride and vinyl pyrrolidone, sold commercially by BASF Aktiengesellschaft, West Germany under the trademark LUVIQUAT at three comonomer ratios, namely at ratios of 95/5, 50/50 and 30/70 methylvinylimidazolium chloride to polyvinylpyrrolidone. These copolymers at all three comonomer ratios have the name Polyquaternium 16. Polymeric conditioners also include cationic cellulosic polymers of hydroxyethyl cellulose reacted with epichlorohydrin and quaternized with trimethylamine, sold under the trademark POLYMER JR in various viscosity grades and molecular sizes by Union Carbide Corporation, Danbury, Conn. These series of polymers are named Polyquaternium 10. Also useful are quaternized copolymers of hydroxyethylcellulose and dimethyldimethylammonium chloride, having the name Polyquaternium-4, sold in varying molecular weights under the trademark CELQUAT by National Starch and Chemical Corporation, Bridgewater, N.J.

Smaller molecule cationic non-polymeric conditioning agents can also be utilized herein. Exemplary small-molecule conditioning agents can include monofunctional or difunctional quaternary ammonium compounds, such as stearyldimethylbenzylammonium chloride, dimethyldi-(hydrogenated tallow)ammonium chloride, and the like. Non-polymeric conditioning agents can also include the quaternary ammonium salts of gluconamide derivatives, such as gamma-gluconamidopropyldimethyl-2-hydroxyethyl-ammonium chloride and minkamidopropyldimethyl-2-hydroxyethylammonium chloride identified respectively by the names Quaternium 22 and Quaternium 26. Details for the preparation of these materials are found in U.S. Pat. Nos. 3,766,267 and 4,012,398, respectively, and the materials are sold under the trademark CERAPHYL by Van Dyk & Co., Belleville, N.J. Also useful are bis-quaternary ammonium compounds which are dimers, such as 2-hydroxy propylene-bis-1,3-(dimethylstearyl ammonium chloride, designated the name, Hydroxypropyl Bisstearyldimonium chloride. The preparation of these and other bis-quat materials is described in U.S. Pat. No. 4,734,277, and such materials are sold under the trademark JORDAQUAT DIMER by Jordan Chemical Company, Folcroft, Pa.

Exemplary unquaternized polymers having tertiary amino nitrogen groups that become quaternized when protonated can include water-soluble proteinaceous quaternary ammonium compounds. Cocodimonium hydrolyzed animal protein, for example, is the name for a chemically-modified quaternary ammonium derivative of hydrolyzed collagen protein having from about 12 to about 18 carbons in at least one aliphatic alkyl group, a weight average molecular weight from about 2500 to about 12,000, and an isoionic point in a range from about 9.5 to about 11.5. This material and structurally related materials are sold under the trademarks CROQUAT and CROTEIN by Croda, Inc., New York, N.Y.

The total amount of the one or more cationic polymers, when included in the hair-treatment composition, is typically about 0.01 to about 15 wt.%, based on the total weight of the composition, including all ranges and subranges therebetween. The total amount of the one or more cationic polymers may be about 0.01 to about 8 wt.%, about 0.01 to about 6 wt.%, about 0.01 to about 5 wt.%, about 0.05 to about 8 wt.%, about 0.05 to about 6 wt.%, about 0.05 to about 5 wt.%, about 0.1 to about 10 wt.%, about 0.1 to about 8 wt.%, about 0.1 to about 6 wt.%, or about 0.1 to about 5 wt.

The term "water-soluble solvent" is interchangeable with the term "water-miscible solvent" and means a compound that is liquid at 25°C and at atmospheric pressure (760 mmHg), and it has a solubility of at least 50% in water under these conditions. The hair-treatment compositions of the instant disclosure may include one or more water-soluble solvents.

Water-soluble solvents include, for example, glycerin, C₁₋₄ alcohols, organic solvents, fatty alcohols, fatty ethers, fatty esters, polyols, glycols, vegetable oils, mineral oils, liposomes, laminar lipid materials, or any combinations thereof. As examples of organic solvents, non-limiting mentions can be made of monoalcohols and polyols such as ethyl alcohol, isopropyl alcohol, propyl alcohol, benzyl alcohol, and phenylethyl alcohol, or glycols or glycol ethers such as, for example, monomethyl, monoethyl and monobutyl ethers of ethylene glycol, propylene glycol or ethers thereof such as, for example, monomethyl ether of propylene glycol, butylene glycol, hexylene glycol, dipropylene glycol as well as alkyl ethers of diethylene glycol, for example monoethyl ether or monobutyl ether of diethylene glycol. Other suitable examples of organic solvents are ethylene glycol, propylene glycol, butylene glycol, hexylene glycol, propane diol, and glycerin. The organic solvents can be volatile or non-volatile compounds.

Further non-limiting examples of water-soluble solvents which may be used include alkanediols (polyhydric alcohols) such as glycerin, 1,2,6-hexanetriol, trimethylolpropane, ethylene glycol, propylene glycol, diethylene glycol, triethylene glycol, tetraethylene glycol, pentaethylene glycol, dipropylene glycol, 2-butene-1,4-diol, 2-ethyl-1,3-hexanediol, 2-methyl-2,4-pentanediol, (CAPRYLYL GLYCOL caprylyl glycol), 1,2-hexanediol, 1,2-pentanediol, and 4-methyl-1,2-pentanediol; alkyl alcohols having 1 to 4 carbon atoms such as ethanol, methanol, butanol, propanol, and isopropanol; glycol ethers such as ethylene glycol monomethyl ether, ethylene glycol monoethyl ether, ethylene glycol monobutyl ether, ethylene glycol monomethyl ether acetate, diethylene glycol monomethyl ether, diethylene glycol monoethyl ether, diethylene glycol mono-n-propyl ether, ethylene glycol mono-iso-propyl ether, diethylene glycol mono-iso-propyl ether, ethylene glycol mono-n-butyl ether, ethylene glycol mono-t-butyl ether, diethylene glycol mono-t-butyl ether, 1-methyl-1-methoxybutanol, propylene glycol monomethyl ether, propylene glycol monoethyl ether, propylene glycol mono-t-butyl ether, propylene glycol mono-n-propyl ether, propylene glycol mono-iso-propyl ether, dipropylene glycol monomethyl ether, dipropylene glycol monoethyl ether, dipropylene glycol mono-n-propyl ether, and dipropylene glycol mono-iso-propyl ether; 2-pyrrolidone, N-methyl-2-pyrrolidone, 1,3-dimethyl-2-imidazolidinone, formamide, acetamide, dimethyl sulfoxide, sorbit, sorbitan, acetine, diacetine, triacetine, sulfolane, and a mixture thereof.

In some cases, the water-soluble solvent may be selected from the group consisting of one or more glycols, C₁₋₄ alcohols, glycerin, and a mixture thereof. In some cases, the water-soluble solvent is selected from the group consisting of hexylene glycol, proplene glycol, caprylyl glycol, glycerin, isopropyl alcohol, and a mixture thereof.

Polyhydric alcohols are useful. Examples of polyhydric alcohols include glycerin, ethylene glycol, diethylene glycol, triethylene glycol, propylene glycol, dipropylene glycol, tripropylene glycol, 1,3-butanediol, 2,3-butanediol, 1,4-butanediol, 3-methyl-1,3-butanediol, 1,5-pentanediol, tetraethylene glycol, 1,6-hexanediol, 2-methyl-2,4-pentanediol, polyethylene glycol, 1,2,4-butanetriol, 1,2,6-hexanetriol, and a mixture thereof.

Polyol compounds may also be used. Non-limiting examples include the aliphatic diols, such as 2-ethyl-2-methyl-1,3-propanediol, 3,3-dimethyl-1,2-butanediol, 2,2-diethyl-1,3-propanediol, 2-methyl-2-propyl-1,3-propanediol, 2,4-dimethyl-2,4-pentanediol, 2,5-dimethyl-2,5-hexanediol, 5-hexene-1,2-diol, and 2-ethyl-1,3-hexanediol, and a mixture thereof.

The total amount of the water-soluble solvents (which is separate than the water in the compositions) may vary, but in some cases are about 0.01 to about 50 wt.%, based on the total weight of the composition. The total amount of the water-soluble solvents (separate from the water in the compositions), is about 0.01 to about 40 wt.%, about 0.01 to about 30 wt.%, about 0.01 to about 20 wt.%, about 0.01 to about 10 wt.%, about 0.01 to about 5 wt.%, 0.1 to about 40 wt.%, about 0.1 to about 30 wt.%, about 0.1 to about 20 wt.%, about 0.1 to about 10 wt.%, or about 0.1 to about 5 wt.%.

The hair-treatment compositions may contain one or more thickeners (also referred to as thickening agents or viscosity modifying agents). Classes of such agents include, but are not limited to, viscous liquids, such as polyethylene glycol, semisynthetic polymers, such as semisynthetic cellulose derivatives, synthetic polymers, such as carbomers, poloxamers, and acrylates/beheneth-25 methacrylate copolymer, acrylates copolymer, polyethyleneimines (e.g., PEI-10), naturally occurring polymers, such as acacia, tragacanth, alginates (e.g., sodium alginate), carrageenan, vegetable gums, such as xanthan gum, petroleum jelly, waxes, particulate associate colloids, such as bentonite, colloidal silicon dioxide, and microcrystalline cellulose, surfactants, such as PPG-2 hydroxyethyl coco/isostearamide, emulsifiers, such as disteareth-75 IPDI, and salts, such as sodium chloride, starches, such as hydroxypropyl starch phosphate, potato starch (modified or unmodified), celluloses such as hydroxyethylcellulose, guars such as hydroxypropyl guar, and a mixture thereof.

In some cases, the thickening agents may include one or more associative thickening polymers such as anionic associative polymers, amphoteric associative polymers, cationic associative polymers, nonionic associative polymers, and a mixture thereof. A non-limiting example of an amphoteric associative polymer is acrylates/beheneth-25methacrylate copolymer, sold under the tradename NOVETHIX L-10 (Lubrizol). Non-limiting examples of anionic associative polymers include INCI name: acrylates copolymer, sold under the tradename CARBOPOL Aqua SF-1 (Lubrizol), INCI name: acrylates crosspolymer-4, sold under the tradename CARBOPOL Aqua SF-2 (Lubrizol), and a mixture thereof. The associative thickening polymers, for instance, the acrylates copolymer and/or the acrylates crosspolymer-4, may be neutralized in water or an aqueous solution with a neutralizing agent before the polymer is added into a hair-treatment composition. In some cases, associative thickening polymers may be useful in anionic surfactant-free hair-treatment compositions, in particular, anionic surfactant free conditioning shampoos. For example, the anionic surfactant-free conditioning shampoos may include one or more anionic associative polymers.

The total amount of the one or more thickening agents may vary, but in some cases is about 0.1 to about 15 wt.%, about 0.1 to about 10 wt.%, about 0.1 to about 8 wt.%, about 0.1 to about 6 wt.%, about 0.1 to about 5 wt.%, about 0.5 to about 10 wt.%, about 0.5 to about 8 wt.%, about 0.5 to about wt.%, about 0.5 to about 5 wt.%, about 1 to about 10 wt.%, about 1 to about 8 wt.%, about 1 to about 6 wt.%, or about 1 to about 5 wt.%, based on the total weight of the composition.

One or more preservatives may be included in the hair-treatment compositions described herein for treating hair. Suitable preservatives include, but are not limited to, glycerin containing compounds (e.g., glycerin or ethylhexylglycerin or phenoxyethanol), benzyl alcohol, parabens (methylparaben, ethylparaben, propylparaben, butylparaben, isobutylparaben, etc.), sodium benzoate, benzoic acid, chlorhexidine digluconate, ethylenediamine-tetraacetic acid (EDTA), potassium sorbate, and/or grapefruit seed extract, or a mixture thereof. Other preservatives are known in the cosmetics industries and include salicylic acid, DMDM Hydantoin, Formaldahyde, Chlorphenism, Triclosan, Imidazolidinyl Urea, Diazolidinyl Urea, Sorbic Acid, Methylisothiazolinone, Sodium Dehydroacetate, Dehydroacetic Acid, Quaternium-15, Stearalkonium Chloride, Zinc Pyrithione, Sodium Metabisulfite, 2-Bromo-2-Nitropropane, Chlorhexidine Digluconate, Polyaminopropyl biguanide, Benzalkonium Chloride, Sodium Sulfite, Sodium Salicylate, Citric Acid, Neem Oil, Essential Oils (various), Lactic Acid, Vitamin E (tocopherol), and a mixture thereof. In some cases, the hair-treatment compsitions may include one or more preservatives selected from the group consisting of sodium benzoate, benzoic acid, chlorhexidine digluconate, chlorhexidine dihydrochloride, salicylic acid, phenoxyethanol, methyl paraben, and a mixture thereof.

The total amount of the one or more preservatives, when present, may vary. In some cases, the total amount of the one or more preservatives is about 0.01 to about 5 wt.%, about 0.01 to about 4 wt.%, about 0.15 to about 1 wt.%, or about 1 to about 3 wt.%, based on the total weight of the composition.

The hair-treatment compositions may be free or essentially free of taurate surfactants and salts thereof. For example, the compositions may be free or essentially free of taurate surfactants and salts thereof, of the following formula:

R₁CO-NR₂-CH₂CH₂SO₃M,

wherein R₁ denotes a saturated or unsaturated hydrocarbon group with an average number of carbon atoms of 7-19; R₂ denotes hydrogen or an alkyl group with an average number of carbon atoms of 1-3; and M denotes an alkali metal, alkali earth metal, ammonium, or organic amine or derivative. Specific examples include N-methyl cocoyl taurate and sodium cocoyl taurate. In some instances, shampoos of the disclosure are free or essentially free of taurate surfactants and salts thereof.

The hair-treatment compositions may be free or essentially free of polyethylene glycol (PEG) and/or derivatives thereof and may be free or essentially free of propylene glycol (PPG) and/or derivatives thereof. For example, the compositions may be free or essentially free of polyethylene glycols having a molecular weight of 200-10,000, or polyethylene glycols having a molecular weight of 200-1,000. In some cases, shampoos of the disclosure are free or essentially free of polyethylene glycol (PEG) and/or derivatives thereof or free or essentially free of propylene glycol (PPG) and/or derivatives thereof. Furthermore, PEGylated surfactants may also be excluded from the hair-treatment compositions and/or booster compositions. Non-limiting examples of PEGylated surfactants include ethoxylated fatty esters.

The pH of the booster compositions can be acidic or alkaline, preferably the pH ranges from 2 to 11. In a more preferred way, the booster composition is acidic, that is to say that the pH is less than 7, preferably ranges from 2.5 to 6.5, even more preferably from 3 to 5.5.

When the hair-treatment composition is a shampoo, the kits and methods of the disclosure can be used in methods for cleansing the hair. When the hair-treatment composition is a conditioner or a rinse-out masque, the kit and methods of the disclosure can be used in methods for conditioning the hair. When the compositions are shampoos, conditioners, or rinse-out masques, the methods typically involve applying a sufficient amount of the composition to the hair followed by rinsing the composition from the hair with water.

The kits and methods are particularly useful in methods for repairing, strengthening, and protecting hair (restructuring hair). Such methods include single treatments and multiple treatments, *e.g*., repeatedly treating the hair with the kits and/or methods. The kits and methods may be repeated daily, every-other-day, three or four times per week, once or twice per week, etc. The repeated treatments may be carried out for a particular period of time, for example, for one week, two weeks, one month, three months, six months, or longer.

The hair-treatment compositions may be packaged in a variety of different containers, such as, for example, a ready-to-use container. Non-limiting examples of useful packaging include tubes, jars, caps, unit dose packages, and bottles, including squeezable tubes and bottles. The packaging may be configured so that it can be attached to a wall, such as a wall in a bathroom, including walls of a shower or tub. For example, the packaging can be a container that is configured to attach to a wall, such that when pressure is applied to the container, the composition contained therein is expelled from one or more openings in the bottom of the container. This type of packing and configuration is convenient for consumers.

The hair-treatment compositions can be in a variety of forms. For example, in many instances, the hair-treatment compositions are in the form of a liquid, gel, lotion, and/or spray; For example, in many instances the hair-treatment compositions are in the form of emulsions, microémulsions, nanoémulsions, or dispersions, and in some instances, the hair-treatment compositions are not in the form of a solid or a paste. Typically, the compositions have a melting temperature of less than 40°C, less than 35°C, less than 30°C, less than 25°C, less than 20°C, less than 15°C, or less than 10°C. In this context, the composition may be formulated, for example, into products such as shampoos including sulfate-free and conditioning shampoos; conditioners; rinse-out masques; leave-in hair products; lotions; gels; emulsions; and general hair nourishing products.

More exhaustive but non-limiting lists of components useful in the hair-treatment compositions disclosed herein are provided below.

### Surfactants

### Cationic Surfactants

The term "cationic surfactant" means a surfactant that is positively charged when it is contained in the composition according to the dislcosure. This surfactant may bear one or more positive permanent charges or may contain one or more functions that are cationizable in the composition according to the disclosure.

Non-limiting examples of cationic surfactants include behenalkonium chloride, benzethonium chloride, cetylpyridinium chloride, behentrimonium chloride, lauralkonium chloride, cetalkonium chloride, cetrimonium bromide, cetrimonium chloride, cethylamine hydrofluoride, chlorallylmethenamine chloride (Quaternium-15), distearyldimonium chloride (Quaternium-5), dodecyl dimethyl ethylbenzyl ammonium chloride (Quaternium-14), Quaternium-22, Quaternium-26, Quaternium-18 hectorite, dimethylaminoethylchloride hydrochloride, cysteine hydrochloride, diethanolammonium POE (10) oletyl ether phosphate, diethanolammonium POE (3)oleyl ether phosphate, tallow alkonium chloride, dimethyl dioctadecylammoniumbentonite, stearalkonium chloride, domiphen bromide, denatonium benzoate, myristalkonium chloride, laurtrimonium chloride, ethylenediamine dihydrochloride, guanidine hydrochloride, pyridoxine HCl, iofetamine hydrochloride, meglumine hydrochloride, methylbenzethonium chloride, myrtrimonium bromide, oleyltrimonium chloride, polyquaternium-1, procainehydrochloride, stearalkonium bentonite, stearalkoniumhectonite, stearyl trihydroxyethyl propylenediamine dihydrofluoride, tallowtrimonium chloride, and hexadecyltrimethyl ammonium bromide.

The cationic surfactant(s) may be chosen from optionally polyoxyalkylenated, primary, secondary or tertiary fatty amines, or salts thereof, and quaternary ammonium salts, and a mixture thereof.

The fatty amines generally comprise at least one C₈-C₃₀ hydrocarbon-based chain.

Examples of quaternary ammonium salts that may especially be mentioned include: those corresponding to the general formula (III) below: 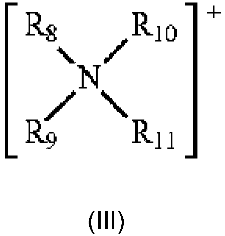 in which the groups R₈ to R₁₁, which may be identical or different, represent a linear or branched, saturated or unsaturated aliphatic group comprising from 1 to 30 carbon atoms, or an aromatic group such as aryl or alkylaryl, at least one of the groups R₈ to R₁₁ denoting a group comprising from 8 to 30 carbon atoms and preferably from 12 to 24 carbon atoms. The aliphatic groups may comprise heteroatoms especially such as oxygen, nitrogen, sulfur and halogens. The aliphatic groups are chosen, for example, from C₁-C₃₀ alkyl, C₂-C₃₀ alkenyl, C₁-C₃₀ alkoxy, polyoxy(C₂-C₆)alkylene, C₁-C₃₀ alkylamide, (C₁₂-C₂₂)alkylamido(C₂-C₆)alkyl, (C₁₂-C₂₂)alkyl acetate and C₁-C₃₀ hydroxyalkyl groups; X⁻is an anion chosen from the group of halides, phosphates, acetates, lactates, (C₁-C₄)alkyl sulfates, and (C₁-C₄)alkyl- or (C₁-C₄)alkylarylsulfonates.

Among the quaternary ammonium salts of formula (III), those that are preferred are, on the one hand, tetraalkylammonium salts, for instance dialkyldimethylammonium or alkyltrimethylammonium salts in which the alkyl group contains approximately from 12 to 22 carbon atoms, in particular behenyltrimethylammonium, distearyldimethylammonium, cetyltrimethylammonium or benzyldimethylstearylammonium salts, or, on the other hand, oleocetyldimethylhydroxyethylammonium salts, palmitylamidopropyltrimethylammonium salts, stearamidopropyltrimethylammonium salts and stearamidopropyldimethylcetearylammonium salts.

In some cases it is useful to use salts such as the chloride salts of the following compounds:
A. a quaternary ammonium salt of imidazoline, such as, for example, those of formula (IV) below: 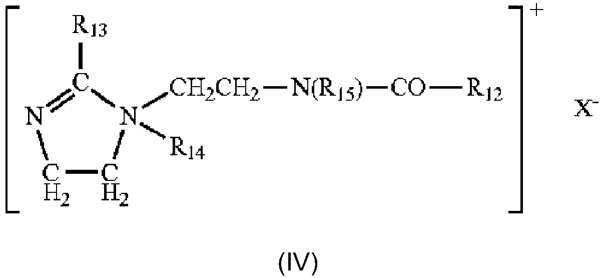 in which R₁₂ represents an alkenyl or alkyl group comprising from 8 to 30 carbon atoms, derived for example from tallow fatty acids, R₁₃ represents a hydrogen atom, a C₁-C₄ alkyl group or an alkyl or alkenyl group comprising from 8 to 30 carbon atoms, R₁₄ represents a C₁-C₄ alkyl group, R₁₅ represents a hydrogen atom or a C₁-C₄ alkyl group, X⁻ is an anion chosen from the group of halides, phosphates, acetates, lactates, alkyl sulfates, alkyl- or alkylaryl-sulfonates in which the alkyl and aryl groups preferably comprise, respectively, from 1 to 20 carbon atoms and from 6 to 30 carbon atoms. R₁₂ and R₁₃ preferably denote a mixture of alkenyl or alkyl groups containing from 12 to 21 carbon atoms, derived for example from tallow fatty acids, R₁₄ preferably denotes a methyl group, and R₁₅ preferably denotes a hydrogen atom. Such a product is sold, for example, under the name REWOQUAT W 75 by the company Evonik;
B. a quaternary diammonium or triammonium salt, in particular of formula (V): 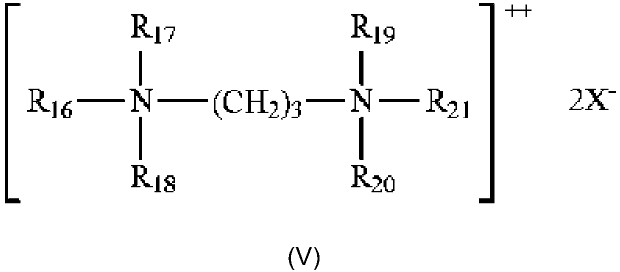
   in which R₁₆ denotes an alkyl radical comprising approximately from 16 to 30 carbon atoms, which is optionally hydroxylated and/or interrupted with one or more oxygen atoms, R₁₇ is chosen from hydrogen or an alkyl radical comprising from 1 to 4 carbon atoms or a group (R₁₆ₐ)(R₁₇ₐ)(R₁₈ₐ)N-(CH₂)₃,
   R₁₆ₐ, R₁₇ₐ, R₁₈ₐ, R₁₈, R₁₉, R₂₀ and R₂₁, which may be identical or different, being chosen from hydrogen and an alkyl radical comprising from 1 to 4 carbon atoms, and X⁻ is an anion chosen from the group of halides, acetates, phosphates, nitrates and methyl sulfates. Such compounds are, for example, FINQUAT CT-P, sold by the company Innospec (Quaternium 89), and FINQUAT CT, sold by the company Innospec (Quaternium 75),
C. a quaternary ammonium salt containing at least one ester function, such as those of formula (VI) below: 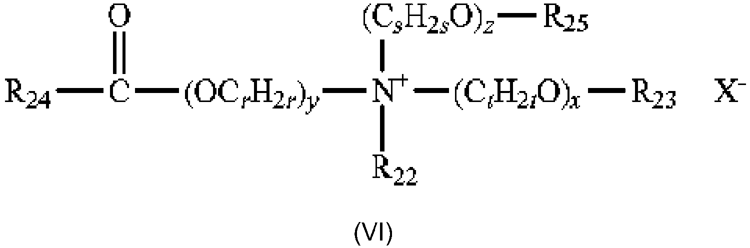 in which:
   R₂₂ is chosen from C₁-C₆ alkyl groups and C₁-C₆ hydroxyalkyl or dihydroxyalkyl groups;
   R₂₃ is chosen from: 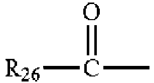 R₂₇, which is a linear or branched, saturated or unsaturated C₁-C₂₂ hydrocarbon-based group, and a hydrogen atom,
   R₂₅ is chosen from: 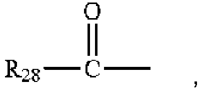 R₂₉, which is a linear or branched, saturated or unsaturated C₁-C₆ hydrocarbon-based group, and a hydrogen atom,
   R₂₄, R₂₆ and R₂₈, which may be identical or different, are chosen from linear or branched, saturated or unsaturated C₇-C₂₁ hydrocarbon-based groups;
   r, s and t, which may be identical or different, are integers ranging from 2 to 6;
   y is an integer ranging from 1 to 10;
   x and z, which may be identical or different, are integers ranging from 0 to 10;
   X⁻ is a simple or complex, organic or mineral anion;
   with the proviso that the sum x+y+z is from 1 to 15, that when x is 0 then Rₙ denotes R₂₇, and that when z is 0 then R₂₅ denotes R₂₉.

The alkyl groups R₂₂ may be linear or branched, and more particularly linear. In some cases, R₂₂ denotes a methyl, ethyl, hydroxyethyl or dihydroxypropyl group, and more particularly a methyl or ethyl group. Advantageously, the sum x+y+z is from 1 to 10.

When R₂₃ is a hydrocarbon-based group R₂₇, it may be long and contain from 12 to 22 carbon atoms, or may be short and contain from 1 to 3 carbon atoms. When R₂₅ is an R₂₉ hydrocarbon-based group, it preferably contains 1 to 3 carbon atoms. Advantageously, R₂₄, R₂₆ and R₂₈, which may be identical or different, are chosen from linear or branched, saturated or unsaturated C₁₁-C₂₁ hydrocarbon-based groups, and more particularly from linear or branched, saturated or unsaturated C₁₁-C₂₁ alkyl and alkenyl groups.

In some cases, x and z, which may be identical or different, have values of 0 or 1. Likewise, in some cases y is equal to 1. In some cases, r, s and t, which may be identical or different, are equal to 2 or 3, and even more particularly are equal to 2.

The anion X⁻ is may be a halide (chloride, bromide or iodide) or an alkyl sulfate, more particularly methyl sulfate. However, use may be made of methanesulfonate, phosphate, nitrate, tosylate, an anion derived from an organic acid, such as acetate or lactate, or any other anion compatible with the ammonium containing an ester function.

The anion X⁻ is even more particularly chloride or methyl sulfate.

Use is made more particularly, in the composition according to the invention, of the ammonium salts of formula (VI) in which:
R₂₂ denotes a methyl or ethyl group,
x and y are equal to 1;
z is equal to 0 or 1;
r, s and t are equal to 2;
R₂₃ is chosen from: 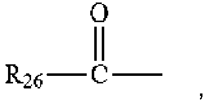 methyl, ethyl or C₁₄-C₂₂ hydrocarbon-based groups, and a hydrogen atom;
R₂₅ is chosen from: 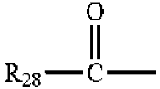 and a hydrogen atom;
R₂₄, R₂₆ and R₂₈, which may be identical or different, are chosen from linear or branched, saturated or unsaturated C₁₃-C₁₇ hydrocarbon-based groups, and preferably from linear or branched, saturated or unsaturated C₁₃-C₁₇ alkyl and alkenyl groups. The hydrocarbon-based groups are advantageously linear.

Mention may be made, for example, of the compounds of formula (VI) such as the diacyloxyethyldimethylammonium, diacylo xyethylhydroxyethylmethylammonium, monoacyloxyethyldihydroxyethylmethylammonium, triacyloxyethylmethylammonium and monoacyloxyethylhydroxyethyldimethylammonium salts (chloride or methyl sulfate in particular), and a mixture thereof. The acyl groups preferably contain 14 to 18 carbon atoms and are obtained more particularly from a plant oil, such as palm oil or sunflower oil. When the compound contains several acyl groups, these groups may be identical or different.

These products are obtained, for example, by direct esterification of triethanolamine, triisopropanolamine, an alkyldiethanolamine or an alkyldiisopropanolamine, which are optionally oxyalkylenated, with C₁₀-C₃₀ fatty acids or with mixtures of C₁₀-C₃₀ fatty acids of plant or animal origin, or by transesterification of the methyl esters thereof. This esterification is followed by quaternization using an alkylating agent such as an alkyl (preferably methyl or ethyl) halide, a dialkyl (preferably methyl or ethyl) sulfate, methyl methanesulfonate, methyl para-toluenesulfonate, glycol chlorohydrin or glycerol chlorohydrin. Such compounds are, for example, sold under the names DEHYQUART by the company BASF, STEPANQUAT by the company Stepan, NOXAMIUM by the company Ceca or REWOQUAT WE 18 by the company Evonik.

The composition according to the invention may contain, for example, a mixture of quaternary ammonium monoester, diester and triester salts with a weight majority of diester salts.

### Anionic Surfactants

The term "anionic surfactant" means a surfactant comprising, as ionic or ionizable groups, only anionic groups. These anionic groups are chosen preferably from the groups CO₂H, CO₂⁻, SO₃H, SO₃⁻, OSO₃H, OSO₃⁻ O₂PO₂H, O₂PO₂H and O₂PO₂²⁻.

The anionic surfactant(s) that may be used may be alkyl sulfates, alkyl ether sulfates, alkylamido ether sulfates, alkylaryl polyether sulfates, monoglyceride sulfates, alkylsulfonates, alkylamide sulfonates, alkylarylsulfonates, alpha-olefin sulfonates, paraffin sulfonates, alkylsulfosuccinates, alkyl ether sulfosuccinates, alkylamide sulfosuccinates, alkyl sulfoacetates, acylsarcosinates, acylglutamates, alkylsulfosuccinamates, acylisethionates and N-acyltaurates, salts of alkyl monoesters and polyglycoside-polycarboxylic acids, acyllactylates, salts of D-galactoside uronic acids, salts of alkyl ether carboxylic acids, salts of alkyl aryl ether carboxylic acids, and salts of alkylamido ether carboxylic acids; or the non-salified forms of all of these compounds, the alkyl and acyl groups of all of these compounds containing from 6 to 24 carbon atoms and the aryl group denoting a phenyl group. Some of these compounds may be oxyethylenated and then preferably comprise from 1 to 50 ethylene oxide units.

The salts of C₆-C₂₄ alkyl monoesters of polyglycoside-polycarboxylic acids may be chosen from C₆-C₂₄ alkyl polyglycoside-citrates, C₆-C₂₄ alkyl polyglycoside-tartrates and C₆-C₂₄ alkyl polyglycoside-sulfo succinates.

When the anionic surfactant(s) are in salt form, they may be chosen especially from alkali metal salts such as the sodium or potassium salt and preferably the sodium salt, ammonium salts, amine salts and in particular amino alcohol salts, or alkaline-earth metal salts such as the magnesium salt.

Examples of amino alcohol salts that may especially be mentioned include monoethanolamine, diethanolamine and triethanolamine salts, monoisopropanolamine, diisopropanolamine or triisopropanolamine salts, 2-amino-2-methyl-1-propanol salts, 2-amino-2-methyl-1,3-propanediol salts and tris(hydroxymethyl)aminomethane salts.Alkali metal or alkaline-earth metal salts and in particular the sodium or magnesium salts may be used.

Use is also made of (C₆-C₂₄)alkyl sulfates, (C₆-C₂₄)alkyl ether sulfates, which are optionally ethoxylated, comprising from 2 to 50 ethylene oxide units, and a mixture thereof, in particular in the form of alkali metal salts or alkaline-earth metal salts, ammonium salts or amino alcohol salts. More preferentially, the anionic surfactant(s) are chosen from (C₁₀-C₂₀)alkyl ether sulfates, and in particular sodium lauryl ether sulfate.

### Amphoteric Surfactants

Amphoteric surfactants useful in the hair-treatment compositions disclosed herein may be chosen from betaines, sultaines, amphoacetates, amphoproprionates, and a mixture thereof. More typically, betaines and amphoproprionates are used, and most typically betaines. Betaines which can be used in the current compositions include those having the formulas below: 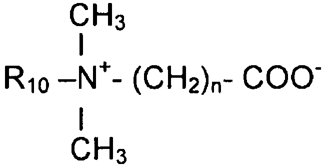 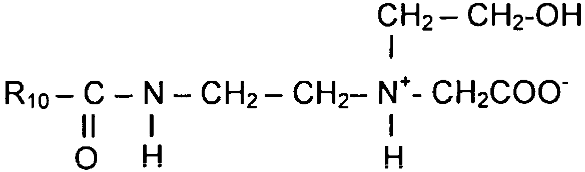 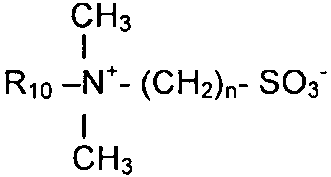 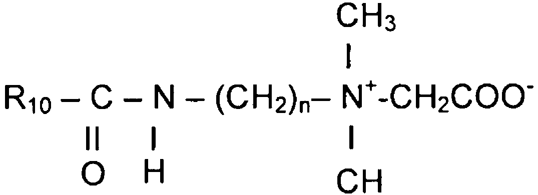 wherein
R¹⁰ is an alkyl group having 8-18 carbon atoms; and
n is an integer from 1 to 3.

Particularly useful betaines include, for example, coco betaine, cocoamidopropyl betaine, lauryl betaine, laurylhydroxy sulfobetaine, lauryldimethyl betaine, cocoamidopropyl hydroxysultaine, behenyl betaine, capryl/capramidopropyl betaine, lauryl hydroxysultaine, stearyl betaine, and a mixture thereof. Typically, the at least one betaine compound is selected from the group consisting of coco betaine, cocoamidopropyl betaine, behenyl betaine, capryl/capramidopropyl betaine, lauryl betaine, and a mixture thereof, and more typically coco betaine and/or cocoamidopropyl betaine.

Hydroxyl sultaines useful in the compositions of the invention include the following 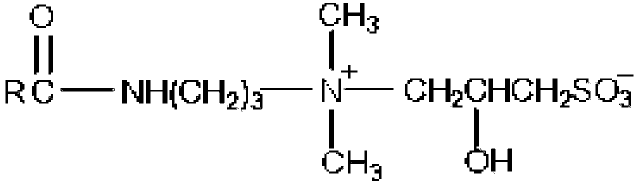 wherein
R is an alkyl group having 8-18 carbon atoms.

Useful alkylamphoacetates include those having the formula 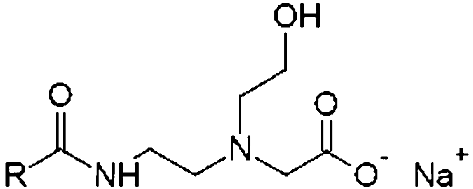 wherein
R is an alkyl group having 8-18 carbon atoms.
useful alkyl amphodiacetates include those having the formula 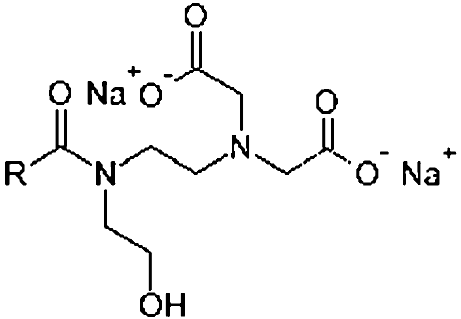
wherein
R is an alkyl group having 8-18 carbon atoms.

The amphoteric surfactants of the present disclosure may be optionally quaternized secondary or tertiary aliphatic amine derivatives, in which the aliphatic group is a linear or branched chain comprising from 8 to 22 carbon atoms, said amine derivatives containing at least one anionic group, for instance a carboxylate, sulfonate, sulfate, phosphate or phosphonate group.

Mention may be made in particular of (C₈-C₂₀)alkylbetaines, (C₈-C₂₀)alkylamido (C₁-C₆)alkylbetaines, sulfobetaines, (C₈-C₂₀)alkylsulfobetaines, (C₈-C₂₀)alkylamido(C₁-C₆)alkylsulfobetaines, (C₈-C₂₀)alkylamphoacetate, (C₈-C₂₀)alkylamphodiacetate, and a mixture thereof.

Among the optionally quaternized secondary or tertiary aliphatic amine derivatives that may be used, mention may also be made of the products of respective structures (A1) and (A2) below:

(A1) Ra-CON(Z)CH₂-(CH₂)m-N+(Rb)(Rc)(CH₂COO-)

in which:
Ra represents a C₁₀-C₃₀ alkyl or alkenyl group derived from an acid Ra-COOH preferably present in hydrolysed coconut oil, a heptyl group, a nonyl group or an undecyl group,
Rb represents a β-hydroxyethyl group,
Rc represents a carboxymethyl group;
m is equal to 0, 1 or 2,
Z represents a hydrogen atom or a hydroxyethyl or carboxymethyl group;

(A2) Ra'-CON(Z)CH₂-(CH₂)m'-N(B)(B')

in which:
B represents -CH₂CH₂OX', with X' representing -CH₂-COOH, CH₂-COOZ', CH₂CH₂-COOH, -CH₂CH₂-COOZ', or a hydrogen atom,
B' represents -(CH₂)z-Y', with z = 1 or 2, and Y' representing COOH, COOZ', CH₂-CHOH-SO₃H or -CH₂-CHOH-SO₃Z',
m' is equal to 0, 1 or 2,
Z represents a hydrogen atom or a hydroxyethyl or carboxymethyl group,
Z' represents an ion resulting from an alkali or alkaline-earth metal, such as sodium, potassium or magnesium; an ammonium ion; or an ion resulting from an organic amine and in particular from an amino alcohol, such as monoethanola-mine, diethanolamine and triethanolamine, monoisopropanolamine, diisopropa-nolamine or triisopropanolamine, 2-amino-2-methyl-1-propanol, 2-amino-2-methyl-1,3-propanediol and tris(hydroxymethyl)aminomethane,
Ra' represents a C₁₀-C₃₀ alkyl or alkenyl group of an acid Ra'COOH preferably pre-sent in hydrolysed linseed oil or coconut oil, an alkyl group, in particular a C₁₇ alkyl group, and its iso form, or an unsaturated C₁₇ group.

Among the compounds corresponding to formula (A2) in which X' represents an hydrogen atom, mention may be made of compounds under the names sodium cocoamphoacetate, sodium lauroamphoacetate, sodium caproamphoacetate and sodium capryloamphoacetate.

Other compounds corresponding to formula (A2) are disodium cocoamphodiace-tate, disodium lauroamphodiacetate, disodium caproamphodiacetate, disodium capryloamphodiacetate, disodium cocoamphodipropionate, disodium lauroam-phodipropionate, disodium caproamphodipropionate, disodium capryloamphodi-propionate, lauroamphodipropionic acid and cocoamphodipropionic acid.

Examples that may be mentioned include the cocoamphodiacetate sold by the company Rhodia under the trade name Miranol^{®} C2M Concentrate, the sodium cocoamphoacetate sold under the trade name Miranol Ultra C 32 and the product sold by the company Chimex under the trade name CHIMEXANE HA.

Use may also be made of the compounds of formula (A3):

(A3) Ra"-NH-CH(Y")-(CH₂)n-C(O)-NH-(CH₂)n'-N(Rd)(Re)

in which:
- Ra" represents a C10-C30 alkyl or alkenyl group of an acid Ra"-C(O)OH preferably present in hydrolysed linseed oil or coconut oil;
- Y" represents the group -C(O)OH, -C(O)OZ", -CH₂-CH(OH)-SO₃H or the group CH₂-CH(OH)-SO₃-Z", with Z" representing a cationic counterion resulting from an alkali metal or alkaline-earth metal, such as sodium, an ammonium ion or an ion resulting from an organic amine;
- Rd and Re represent, independently of each other, a C₁-C₄ alkyl or hydroxyalkyl radical; and
- n and n' denote, independently of each other, an integer ranging from 1 to 3.

Among the compounds corresponding to formula (A3), mention may in particular be made of the compound under the name sodium diethylaminopropylcocoaspartamide.

Preferably, the amphoteric surfactants are chosen from (C₈-C₂₀)alkylbetaines, (C₈-C₂₀)alkylamido(C₁-C₆)alkylbetaines, (C₈-C₂₀)alkylamphoacetates and (C₈-C₂₀)alkylamphodiacetates, and a mixture thereof.

In some cases, the at least one amphoteric surfactant is chosen from (C₈-C₂₀)alkyl betaines, (C₈-C₂₀)alkylamido (C₁-C₆)alkylbetaines, (C₈-C₂₀)alkylamphoacetate, (C₈-C₂₀)alkylamphodiacetate, and their salts, and a mixture thereof. In some cases, the at least one amphoteric surfactant is selected from coco-betaine, cocamidopropylbetaine, sodium cocoamphoacetate, disodium cocoamphodiacetate, and a mixture thereof.

### Non-Ionic Surfactants

Nonionic surfactants are compounds well known in themselves (*see, e.g*., in this regard, "Handbook of Surfactants" by M. R. Porter, Blackie & Son publishers (Glasgow and London), 1991, pp. 116-178), which is incorporated herein by reference in its entirety.

The nonionic surfactant can be, for example, selected from alcohols, alpha-diols, alkylphenols and esters of fatty acids, these compounds being ethoxylated, propoxylated or glycerolated and having at least one fatty chain comprising, for example, from 8 to 18 carbon atoms, it being possible for the number of ethylene oxide or propylene oxide groups to range from 2 to 50, and for the number of glycerol groups to range from 1 to 30. Maltose derivatives may also be mentioned. Non-limiting mention may also be made of copolymers of ethylene oxide and/or of propylene oxide; condensates of ethylene oxide and/or of propylene oxide with fatty alcohols; polyethoxylated fatty amides comprising, for example, from 2 to 30 mol of ethylene oxide; polyglycerolated fatty amides comprising, for example, from 1.5 to 5 glycerol groups, such as from 1.5 to 4; ethoxylated fatty acid esters of sorbitan comprising from 2 to 30 mol of ethylene oxide; ethoxylated oils from plant origin; fatty acid esters of sucrose; fatty acid esters of polyethylene glycol; polyethoxylated fatty acid mono or diesters of glycerol (C₆-C₂₄)alkylpolyglycosides; N-(C₆-C₂₄)alkylglucamine derivatives, amine oxides such as (C₁₀-C₁₄)alkylamine oxides or N-(C₁₀-C₁₄)acylaminopropylmorpholine oxides; and a mixture thereof.

The nonionic surfactants may preferably be chosen from polyoxyalkylenated or polyglycerolated nonionic surfactants. The oxyalkylene units are more particularly oxyethylene or oxypropylene units, or a combination thereof, and are preferably oxyethylene units.

Examples of oxyalkylenated nonionic surfactants that may be mentioned include: [0115] oxyalkylenated (C₈-C₂₄)alkylphenols, saturated or unsaturated, linear or branched, oxyalkylenated C₈-C₃₀ alcohols, saturated or unsaturated, linear or branched, oxyalkylenated C₈-C₃₀ amides, esters of saturated or unsaturated, linear or branched, C₈-C₃₀ acids and of polyethylene glycols, polyoxyalkylenated esters of saturated or unsaturated, linear or branched, C₈-C₃₀ acids and of sorbitol, saturated or unsaturated, oxyalkylenated plant oils, condensates of ethylene oxide and/or of propylene oxide, inter alia, alone or as mixtures.

The surfactants preferably contain a number of moles of ethylene oxide and/or of propylene oxide of between 2 and 100 and most preferably between 2 and 50. Advantageously, the nonionic surfactants do not comprise any oxypropylene units.

In accordance with one preferred embodiment of the invention, the oxyalkylenated nonionic surfactants are chosen from oxyethylenated C₈-C₃₀ alcohols.

Examples of ethoxylated fatty alcohols (or C₈-C₃₀ alcohols) that may be mentioned include the adducts of ethylene oxide with lauryl alcohol, especially those containing from 9 to 50 oxyethylene groups and more particularly those containing from 10 to 25 oxyethylene groups (Laureth-10 to Laureth-25); the adducts of ethylene oxide with behenyl alcohol, especially those containing from 9 to 50 oxyethylene groups (Beheneth-9 to Beheneth-50); the adducts of ethylene oxide with cetearyl alcohol (mixture of cetyl alcohol and stearyl alcohol), especially those containing from 10 to 30 oxyethylene groups (Ceteareth-10 to Ceteareth-30); the adducts of ethylene oxide with cetyl alcohol, especially those containing from 10 to 30 oxyethylene groups (Ceteth-10 to Ceteth-30); the adducts of ethylene oxide with stearyl alcohol, especially those containing from 10 to 30 oxyethylene groups (Steareth-10 to Steareth-30); the adducts of ethylene oxide with isostearyl alcohol, especially those containing from 10 to 50 oxyethylene groups (Isosteareth-10 to Isosteareth-50); and a mixture thereof.

As examples of polyglycerolated nonionic surfactants, polyglycerolated C₈-C₄₀ alcohols are preferably used.

In particular, the polyglycerolated C₈-C₄₀ alcohols correspond to the following formula:

RO-[CH₂-CH(CH₂OH)-O]ₘ-H or RO-[CH(CH₂OH)-CH₂O]ₘ-H

in which R represents a linear or branched C₈-C₄₀ and preferably C₈-C₃₀ alkyl or alkenyl radical, and m represents a number ranging from 1 to 30 and preferably from 1.5 to 10.

As examples of compounds that are suitable in the context of the invention, mention may be made of lauryl alcohol containing 4 mol of glycerol (INCI name: Polyglyceryl-4 Lauryl Ether), lauryl alcohol containing 1.5 mol of glycerol, oleyl alcohol containing 4 mol of glycerol (INCI name: Polyglyceryl-4 Oleyl Ether), oleyl alcohol containing 2 mol of glycerol (INCI name: Polyglyceryl-2 Oleyl Ether), cetearyl alcohol containing 2 mol of glycerol, cetearyl alcohol containing 6 mol of glycerol, oleocetyl alcohol containing 6 mol of glycerol, and octadecanol containing 6 mol of glycerol.

The alcohol may represent a mixture of alcohols in the same way that the value of m represents a statistical value, which means that, in a commercial product, several species of polyglycerolated fatty alcohol may coexist in the form of a mixture.

According to one of the embodiments according to the present invention, the nonionic surfactant may be selected from esters of polyols with fatty acids with a saturated or unsaturated chain containing for example from 8 to 24 carbon atoms, preferably 12 to 22 carbon atoms, and alkoxylated derivatives thereof, preferably with a number of alkyleneoxide of from 10 to 200, and more preferably from 10 to 100, such as glyceryl esters of a C₈-C₂₄, preferably C₁₂-C₂₂, fatty acid or acids and alkoxylated derivatives thereof, preferably with a number of alkyleneoxide of from 10 to 200, and more preferably from 10 to 100; polyethylene glycol esters of a C₈-C₂₄, preferably C₁₂-C₂₂, fatty acid or acids and alkoxylated derivatives thereof, preferably with a number of alkyleneoxide of from 10 to 200, and more preferably from 10 to 100; sorbitol esters of a C₈-C₂₄, preferably C₁₂-C₂₂, fatty acid or acids and alkoxylated derivatives thereof, preferably with a number of alkyleneoxide of from 10 to 200, and more preferably from 10 to 100; sugar (sucrose, glucose, alkylglycose) esters of a C₈-C₂₄, preferably C₁₂-C₂₂, fatty acid or acids and alkoxylated derivatives thereof, preferably with a number of alkyleneoxide of from 10 to 200, and more preferably from 10 to 100; ethers of fatty alcohols; ethers of sugar and a C₈-C₂₄, preferably C₁₂-C₂₂, fatty alcohol or alcohols; and a mixture thereof.

Examples of ethoxylated fatty esters that may be mentioned include the adducts of ethylene oxide with esters of lauric acid, palmitic acid, stearic acid or behenic acid, and a mixture thereof, especially those containing from 9 to 100 oxyethylene groups, such as PEG-9 to PEG-50 laurate; PEG-9 to PEG-50 palmitate; PEG-9 to PEG-50 stearate; PEG-9 to PEG-50 palmitostearate; PEG-9 to PEG-50 behenate; polyethylene glycol 100 EO monostearate; and a mixture thereof.

As glyceryl esters of fatty acids, glyceryl stearate (glyceryl mono-, di- and/or tristearate) (glyceryl stearate) or glyceryl ricinoleate and a mixture thereof can in particular be cited.

As glyceryl esters of C₈-C₂₄ alkoxylated fatty acids, polyethoxylated glyceryl stearate (glyceryl mono-, di- and/or tristearate) such as PEG-20 glyceryl stearate can for example be cited.

Mixtures of these surfactants, such as for example the product containing glyceryl stearate and PEG-100 stearate, marketed under the name ARLACEL 165 by Croda, and a product containing glyceryl stearate (glyceryl mono- and distearate) and potassium stearate, can also be used.

The sorbitol esters of C₈-C₂₄ fatty acids and alkoxylated derivatives thereof can be selected from sorbitan palmitate, sorbitan trioleate and esters of fatty acids and alkoxylated sorbitan containing for example from 20 to 100 EO, such as for example polyethylene sorbitan trioleate (polysorbate 85) or the compounds marketed under the trade names Tween 20 or Tween 60 by Croda.

As esters of fatty acids and glucose or alkylglucose, in particular glucose palmitate, alkylglucose sesquistearates such as methylglucose sesquistearate, alkylglucose palmitates such as methylglucose or ethylglucose palmitate, methylglucoside fatty esters and more specifically the diester of methylglucoside and oleic acid (Methyl glucose dioleate), the mixed ester of methylglucoside and the mixture oleic acid/hydroxystearic acid (Methyl glucose dioleate/hydroxystearate), the ester of methylglucoside and isostearic acid (Methyl glucose isostearate), the ester of methylglucoside and lauric acid (Methyl glucose laurate), the mixture of monoester and diester of methylglucoside and isostearic acid (Methyl glucose sesqui-isostearate), the mixture of monoester and diester of methylglucoside and stearic acid (Methyl glucose sesquistearate) and in particular the product marketed under the name Glucate SS by Lubrizol, and a mixture thereof can be cited.

As ethoxylated ethers of fatty acids and glucose or alkylglucose, ethoxylated ethers of fatty acids and methylglucose, and in particular the polyethylene glycol ether of the diester of methylglucose and stearic acid with about 20 moles of ethylene oxide (PEG-20 methyl glucose distearate) such as the product marketed under the name GLUCAM E-20 DISTEARATE by Lubrizol, the polyethylene glycol ether of the mixture of monoester and diester of methyl-glucose and stearic acid with about 20 moles of ethylene oxide (PEG-20 methyl glucose sesquistearate) and in particular the product marketed under the name GLUCAMATE SSE-20 by Lubrizol, and a mixture thereof, can for example be cited.

As sucrose esters, saccharose palmito-stearate, saccharose stearate and saccharose monolaurate can for example be cited.

As sugar ethers, alkylpolyglucosides can be used, and for example decylglucoside such as the product marketed under the name MYDOL 10 by Kao Chemicals, the product marketed under the name PLATAREN 2000 by BASF, and the product marketed under the name ORAMIX NS 10 by Seppic, caprylyl/capryl glucoside such as the product marketed under the name ORAMIX CG 110 by Seppic or under the name LUTENSOL GD 70 by BASF, laurylglucoside such as the products marketed under the names PLANTAREN 1200 N and PLANTACARE 1200 by BASF, coco-glucoside such as the product marketed under the name PLANTACARE 818/UP by BASF, cetostearyl glucoside possibly mixed with cetostearyl alcohol, marketed for example under the name MONTANOV 68 by Seppic, under the name TEGO-CARE CG90 by Evonik, arachidyl glucoside, for example in the form of the mixture of arachidyl and behenyl alcohols and arachidyl glucoside marketed under the name MONTANOV 202 by Seppic, cocoylethylglucoside, for example in the form of the mixture (35/65) with cetyl and stearyl alcohols, marketed under the name MONTANOV 82 by Seppic, and a mixture thereof can in particular be cited.

Mixtures of glycerides of alkoxylated plant oils such as mixtures of ethoxylated (200 EO) palm and copra (7 EO) glycerides can also be cited.

It is preferable that the nonionic surfactant be selected from the group consisting of PEG-7 glyceryl cocoate, PEG-20 methylglucoside sesquistearate, PEG-20 glyceryl tri-isostearate, PG-5 dioleate, PG-4 diisostearate, PG-10 isostearate, PEG-8 isostearate, and PEG-60 hydrogenated castor oil.

Mixtures of these oxyethylenated derivatives of fatty alcohols and of fatty esters may also be used.

Preferably, the nonionic surfactant may be a nonionic surfactant with an HLB of 18.0 or less, such as from 4.0 to 18.0, more preferably from 6.0 to 15.0 and furthermore preferably from 9.0 to 13.0. The HLB is the ratio between the hydrophilic part and the lipophilic part in the molecule. This term HLB is well known to those skilled in the art and is described in "The HLB system. A time-saving guide to emulsifier selection" (published by ICI Americas Inc., 1984).

In some case, the nonionic surfactant is a fatty alkanolamide. Non-limiting examples of fatty alkanolamides that may be used include cocamide MEA, cocamide DEA, soyamide DEA, lauramide DEA, oleamide MIPA, stearamide MEA, myristamide DEA, stearamide DEA, oleylamide DEA, tallowamide DEA lauramide MIPA, tallowamide MEA, isostearamide DEA, isostearamide MEA, and a mixture thereof.

### Silicones

Exemplary silicones include, without limitation, cyclic silicones, such as those having 3 to 6, or 3 to 4 or 3 to 5, (or any of 3, 4, 5, or 6) Si--O groups in the cyclic backbone chain (e.g., siloxanes). In some cases, the cyclic silicone is a volatile silicone. In some cases, the cyclic silicone is a low viscosity silicone. Exemplary cyclic silicones include, without limitation, cyclomethicone, cyclotetrasiloxane, cyclopentasiloxane (e.g., Cyclomethicone 5-NF), cyclohexasiloxane and a mixture of cyclohexasiloxane and cyclopenasiloxane (e.g., DOW CORNING 246 Fluid (d6+d5)). Other non-limiting examples of silicones are silicones having side groups or side chains. In some cases, the side groups are hydrophobic. In some cases, the side groups are straight chained, while in other embodiments the side groups are branched. Exemplary side chains include those having 1 to 6, or 2 to 6, or 3 to 6 or 3 to 6 or 5 to 6 carbons or heteroatoms (e.g., O, S, or N) (or any combination thereof). Exemplary linear side chains include, without limitation, methyl, ethyl, propyl, butyl, pentyl, and hexyl. Exemplary branched side chains include, without limitation, isopropyl, isobutyl, and tert-butyl. In one nonlimiting embodiment, the branched side chain is --O--Si(CH₃)₃. Nonlimiting examples of silicones having branched side chains are stearyl dimethicone and phyenyltrimethicone, cetyl dimethicone, caprylyl methicone, PEG/PPG 18/18 dimethicone the structures of which are as follows:

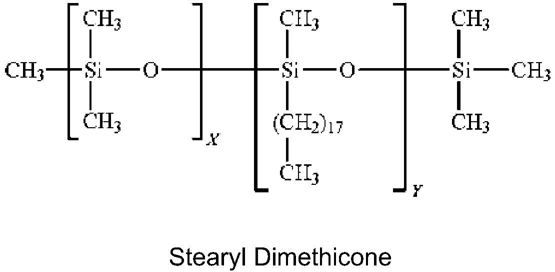

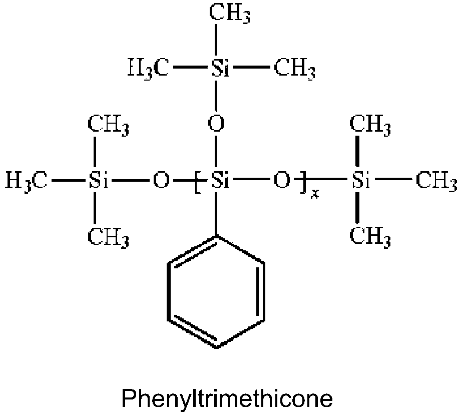

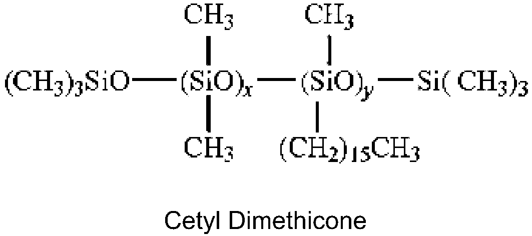

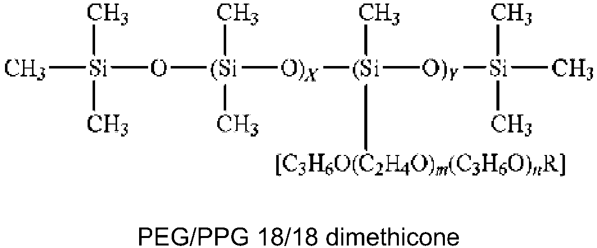

In the above formulas m, n, x, and y may independently be integers of 1 to 100, 1 to 80, 1 to 60, 1 to 50, 1 to 40, 1 to 30, 1 to 20, or 1 to 10. In some cases, the side chains are cyclic. Cyclic side chains include aliphatic side chains and aromatic side chains. A nonlimiting example of a cyclic side chain is phenyl.

With regard to silicones having hydrophilic or polar groups, as described previously, silicones that are repulsive with regard to the hydrophobic chains of the oil are thought to produce more stable foams because they do not inhibit the hydrophobic-hydrophobic interactions of the oil. Exemplary hydrophilic or polar groups include oxygen-containing groups, such as carbonyl groups, hydroxy groups, ether, ester, carboxylic groups, which replace one or more methyl groups. The hydrophilic/polar groups are present alternatively in the main chain of the silicone or in a side chain. Nonlimiting examples of a silicone having a hydrophilic group are PEG/PPG 18/18 dimethicone and dimethiconol, the structures of which are:

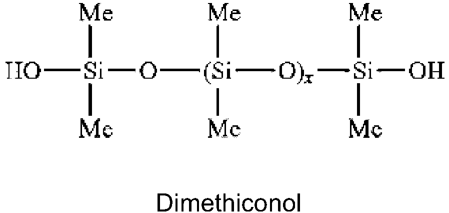

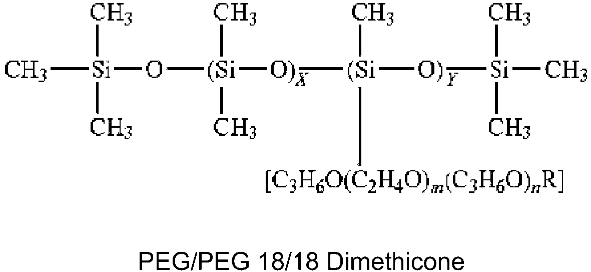

X, y, m, and n are as defined above, and R is a C₁ to C₁₀ alkyl.

Another type of specific non limiting volatile silicone is a volatile short chain linear alkylmethylsilicone fluid. The volatile short chain linear alkylmethylsilicone fluid has the formula:

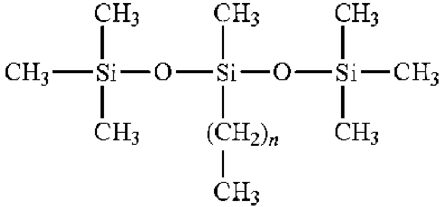

In the above formula, the integer represented by n has a value of five to twelve. Preferably, n has a value of five to eight. Compounds include, for example, 3-hexyl-1,1,1,3,5,5,5,-heptamethyltrisiloxane and 3-octyl-1,1,1,3,5,5,5-heptamethyltrisiloxane.

Yet another type of volatile silicone in accordance with the present invention is a volatile short chain linear phenylmethylsilicone fluid. The volatile short chain linear phenylmethylsilicone fluid has the formula:

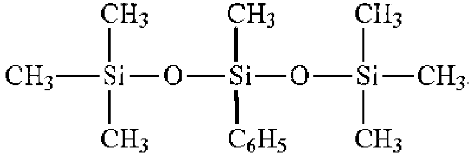

This compound is 3-phenyl-1,1,1,3,4,4,4-heptamethyltrisiloxane. Further volatile silicone fluids useful in the compositions described herein include, without limitation, are decamethylcyclopentasiloxane (DMCPS) which has a molecular weight of about 370, a refractive index of 1.40, and the formula [(Me₂)SiO]₅; the compound 3-hexyl-1,1,1,3,5,5,5-heptamethyltrisiloxane (HHMTS) which has a molecular weight of about 306, and a refractive index of 1.41; and the compound 3-phenyl-1,1,1,3,5,5,5-heptamethyltrisiloxane (PHMTS) which has a molecular weight of about 298 and a refractive index of 1.45.

As amino silicone that may be used in the scope of the instant disclosure, the following can be cited:
a) polysiloxanes corresponding to formula (A): 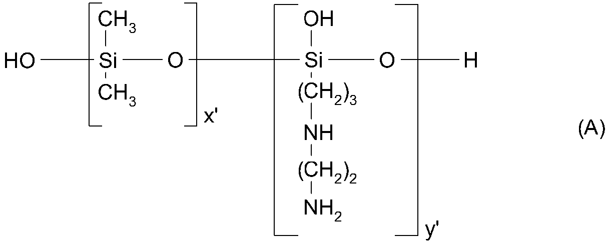 in which x' and y' are integers such that the weight-average molecular weight (Mw) is comprised between about 5000 and 500 000
b) amino silicones correspondingto formula (B):

   R'ₐG₃₋ₐ-Si(OSiG₂)n-(OSiGbR'_{2-b})m-O-SiG₃₋ₐ-R'ₐ (B)

   in which:
   - G, which may be identical or different, designate a hydrogen atom, or a phenyl, OH or C₁-C₈ alkyl group, for example methyl, or C₁-C₈ alkoxy, for example methoxy,
   - a, which may be identical or different, denote the number 0 or an integer from 1 to 3, in particular 0;
   - b denotes 0 or 1, and in particular 1;
   - m and n are numbers such that the sum (n + m) ranges from 1 to 2000 and in particular from 50 to 150, it being possible for n to denote a number from 0 to 1999 and in particular from 49 to 149, and for m to denote a number from 1 to 2000 and in particular from 1 to 10;
   - R', which may be identical or different, denote a monovalent radical having formula -CqH₂qL in which q is a number ranging from 2 to 8 and L is an optionally quaternized amino group chosen from the following groups:

      -NR"-Q-N(R")₂

      -N(R")₂

      -N+(R")₃ A-

      -N+H(R")₂ A-

      -N+H₂(R") A-

      -N(R")-Q-N+R"H₂ A-

      -NR"-Q-N+ (R")₂H A-

      -NR"-Q-N+ (R")₃ A-,
   in which R", which may be identical or different, denote hydrogen, phenyl, benzyl, or a saturated monovalent hydrocarbon-based radical, for example a C₁-C₂₀ alkyl radical; Q denotes a linear or branched CrH₂ᵣ group, r being an integer ranging from 2 to 6, preferably from 2 to 4; and A- represents a cosmetically acceptable ion, in particular a halide such as fluoride, chloride, bromide or iodide.

A group of amino silicones corresponding to this definition (B) is represented by the silicones called "trimethylsilylamodimethicone" having formula (C): 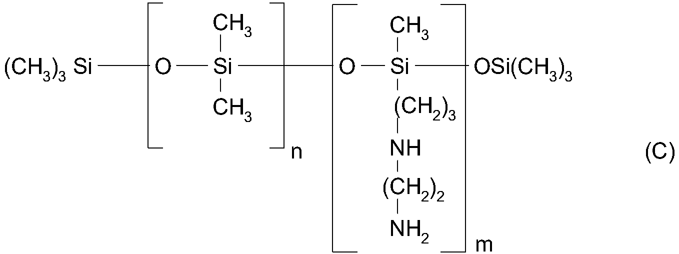 in which n and m have the meanings given above, in formula B.

Another group of amino silicones corresponding to this definition is represented by silicones having the following formulae (D) or (E): 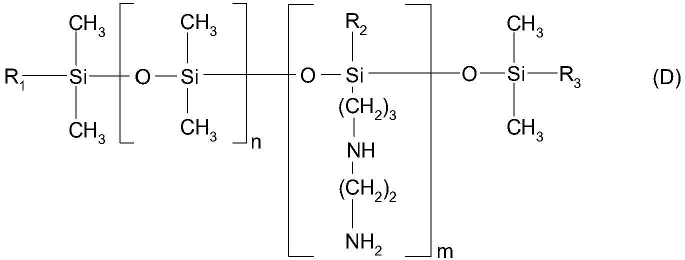 in which:
- m and n are numbers such that the sum (n + m) can range from 1 to 1000, in particular from 50 to 250 and more particularly from 100 to 200, it being possible for n to denote a number from 0 to 999 and in particular from 49 to 249, and more particularly from 125 to 175, and for m to denote a number from 1 to 1000 and in particular from 1 to 10, and more particularly from 1 to 5;
- R₁, R₂, R₃, which may be identical or different, represent a hydroxy or C₁-C₄ alkoxy radical, where at least one of the radicals R₁ to R₃ denotes an alkoxy radical.

The alkoxy radical is preferably a methoxy radical.

The hydroxy/alkoxy mole ratio ranges preferably from 0.2:1 to 0.4:1 and preferably from 0.25:1 to 0.35:1 and more particularly equals 0.3:1.

The weight-average molecular weight (Mw) of the silicone ranges preferably from 2000 to 1 000 000, more particularly from 3500 to 200 000. 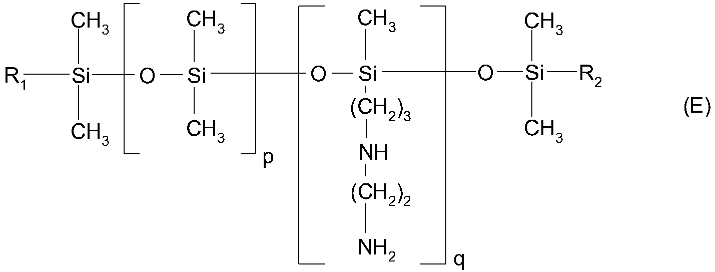 in which:
- p and q are numbers such that the sum (p + q) ranges from 1 to 1000, particularly from 50 to 350, and more particularly from 150 to 250; it being possible for p to denote a number from 0 to 999 and in particular from 49 to 349, and more particularly from 159 to 239 and for q to denote a number from 1 to 1000, in particular from 1 to 10, and more particularly from 1 to 5;
- R₁, R₂, which are different, represent a hydroxy or C₁-C₄ alkoxy radical, where at least one of the radicals R₁ or R₂ denotes an alkoxy radical.

The alkoxy radical is preferably a methoxy radical.

The hydroxy/alkoxy mole ratio ranges generally from 1:0.8 to 1:1.1 and preferably from 1:0.9 to 1:1 and more particularly equals 1:0.95.

The weight-average molecular weight (Mw) of the silicone ranges preferably from 2000 to 200 000, even more particularly 5000 to 100 000 and more particularly from 10 000 to 50 000.

Commercial products corresponding to these silicones having structure (D) or (E) may include in their composition one or more other amino silicones whose structure is different than formulae (D) or (E).

A product containing amino silicones having structure (D) is sold by Wacker under the name Belsil^{®} ADM 652.

A product containing amino silicones having structure (E) is sold by Wacker under the name Fluid WR 1300^{®}.

When these amino silicones are used, one particularly advantageous embodiment consists in using them in the form of an oil-in-water emulsion. The oil-in-water emulsion may comprise one or more surfactants. The surfactants may be of any nature but are preferably cationic and/or nonionic. The number-average size of the silicone particles in the emulsion generally ranges from 3 nm to 500 nanometres. Preferably, in particular as amino silicones having formula (E), microemulsions are used whose average particle size ranges from 5 nm to 60 nanometres (limits included) and more preferably from 10 nm to 50 nanometres (limits included). Accordingly, according to the invention the microemulsions of amino silicone having formula (E) sold as Finish CT 96 E^{®} or SLM 28020^{®} by Wacker can be used.

Another group of amino silicones corresponding to this definition is represented by the following formula (F): 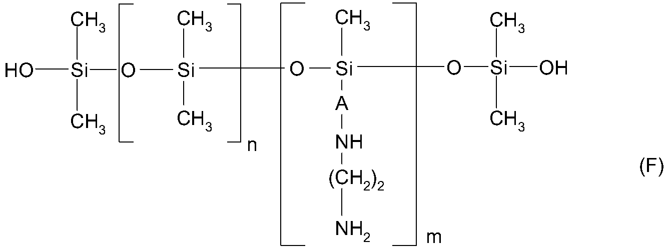 in which:
- m and n are numbers such that the sum (n + m) ranges from 1 to 2000 and in particular from 50 to 150, it being possible for n to denote a number from 0 to 1999 and in particular from 49 to 149, and form to denote a number from 1 to 2000 and in particular from 1 to 10;
- A denotes a linear or branched alkylene radical containing from 4 to 8 carbon atoms and preferably 4 carbon atoms. This radical is preferably linear.

The weight-average molecular weight (Mw) of these amino silicones ranges preferably from 2000 to 1 000 000 and even more particularly from 3500 to 200 000.

A preferred silicone of formula (F) is amodimethicone (INCI name) sold under the tradename XIAMETER^{®} MEM-8299 Cationic Emulsion by Dow Corning.

Another group of amino silicones corresponding to this definition is represented by the following formula (G): 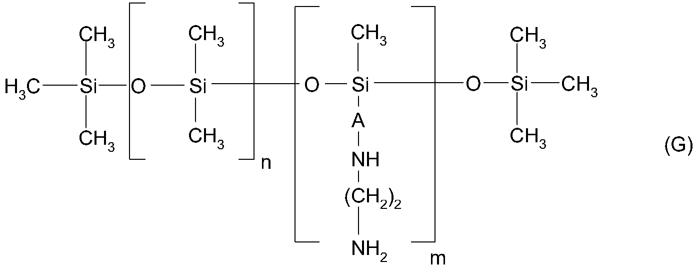 in which:
- m and n are numbers such that the sum (n + m) ranges from 1 to 2000 and in particular from 50 to 150, it being possible for n to denote a number from 0 to 1999 and in particular from 49 to 149, and form to denote a number from 1 to 2000 and in particular from 1 to 10;
- A denotes a linear or branched alkylene radical containing from 4 to 8 carbon atoms and preferably 4 carbon atoms. This radical is preferably branched.

The weight-average molecular weight (Mw) of these amino silicones ranges preferably from 500 to 1 000 000 and even more particularly from 1000 to 200 000.

A silicone having this formula is for example DC2-8566 Amino Fluid by Dow Corning.
c) amino silicones corresponding to formula (H): 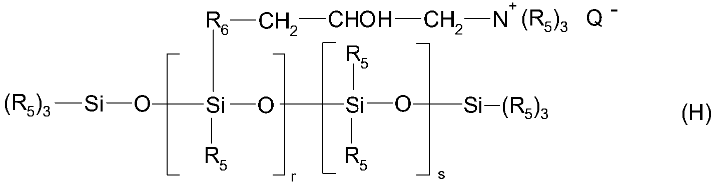
in which:
   - R₅ represents a monovalent hydrocarbon-based radical containing from 1 to 18 carbon atoms, and in particular a C₁-C₁₈ alkyl or C₂-C₁₈ alkenyl radical, for example methyl;
   - R₆ represents a divalent hydrocarbon-based radical, in particular a C₁-C₁₈ alkylene radical or a divalent C₁-C₁₈, for example C₁-C₈, alkylenoxy radical linked to the Si via an SiC bond;
   - Q- is an anion such as a halide ion, in particular chloride, or an organic acid salt (for example acetate);
   - r represents a mean statistical value from 2 to 20 and in particular from 2 to 8;
   - s represents a mean statistical value from 20 to 200 and in particular from 20 to 50.

Such amino silicones are described more particularly in patent US 4 185 087.
d) quaternary ammonium silicones having formula (I): 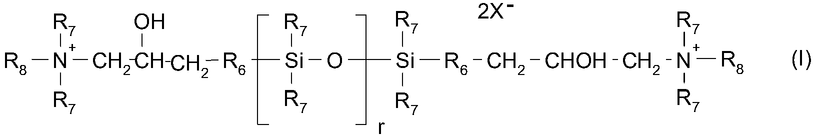
in which:
   - R₇, which may be identical or different, represent a monovalent hydrocarbon-based radical containing from 1 to 18 carbon atoms, and in particular a C₁-C₁₈ alkyl radical, a C₂-C₁₈ alkenyl radical or a ring containing 5 or 6 carbon atoms, for example methyl;
   - R₆ represents a divalent hydrocarbon-based radical, in particular a C₁-C₁₈ alkylene radical or a divalent C₁-C₁₈, for example C₁-C₈, alkylenoxy radical linked to the Si via an SiC bond;
   - R₈, which may be identical or different, represent a hydrogen atom, a monovalent hydrocarbon-based radical containing from 1 to 18 carbon atoms, and in particular a C₁-C₁₈ alkyl radical, a C₂-C₁₈ alkenyl radical or a -R₆-NHCOR₇ radical;
   - X- is an anion such as a halide ion, in particular chloride, or an organic acid salt (for example acetate);
   - r represents a mean statistical value from 2 to 200 and in particular from 5 to 100;

These silicones are described, for example, in patent application EP-A 0 530 974.
e) amino silicones having formula (J): 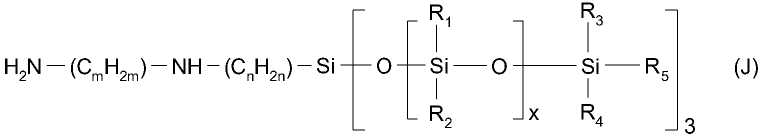
in which:
   - R₁, R₂, R₃ and R₄, which may be identical or different, denote a C₁-C₄ alkyl radical or a phenyl group;
   - R₅ denotes a C₁-C₄ alkyl radical or a hydroxyl group;
   - n is an integer ranging from 1 to 5;
   - m is an integer ranging from 1 to 5;
and in which x is chosen such that the amine number is between 0.01 and 1 meq/g;
f) multiblockpolyoxyalkylenated amino silicones, of type (AB)n, A being a polysiloxane block and B being a polyoxyalkylenated block containing at least one amine group.

Said silicones are preferably constituted of repeating units having the following general formulae:

[-(SiMe₂O)ₓSiMe₂-R-N(R")-R'-O(C₂H₄O)ₐ(C₃H₆O)_{b}-R'-N(H)-R-]

or alternatively

[-(SiMe₂O)ₓSiMe₂-R-N(R")-R'-O(C₂H₄O)ₐ(C₃H₆O)_{b}-]

in which:
- a is an integer greater than or equal to 1, preferably ranging from 5 to 200, more particularly ranging from 10 to 100;
- b is an integer comprised between 0 and 200, preferably ranging from 4 to 100, more particularly between from 5 and 30;
- x is an integer ranging from 1 to 10 000, more particularly from 10 to 5000;
- R" is a hydrogen atom or a methyl;
- R, which may be identical or different, represent a divalent linear or branched C₂-C₁₂ hydrocarbon-based radical, optionally including one or more heteroatoms such as oxygen; preferably, R denotes an ethylene radical, a linear or branched propylene radical, a linear or branched butylene radical, or a - CH₂CH₂CH₂OCH(OH)CH₂- radical; preferentially R denotes a - CH₂CH₂CH₂OCH(OH)CH₂- radical;
- R', which may be identical or different, represent a divalent linear or branched C₂-C₁₂ hydrocarbon-based radical, optionally including one or more heteroatoms such as oxygen; preferably, R' denotes an ethylene radical, a linear or branched propylene radical, a linear or branched butylene radical, or a - CH₂CH₂CH₂OCH(OH)CH₂- radical; preferentially R' denotes -CH(CH₃)-CH₂-.

The siloxane blocks preferably represent between 50 and 95 mol% of the total weight of the silicone, more particularly from 70 to 85 mol%.

The amine content is preferably between 0.02 and 0.5 meq/g of copolymer in a 30% solution in dipropylene glycol, more particularly between 0,05 and 0,2.

The weight-average molecular weight (Mw) of the silicone is preferably comprised between 5000 and 1 000 000, more particularly between 10 000 and 200 000.

Mention may be made especially of the silicones sold under the names Silsoft^{™} A-843 or Silsoft^{™} A+ by Momentive.
g) the alkylamino silicones corresponding to formula (K) below: 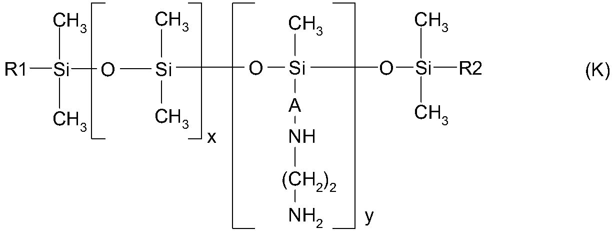
in which:
   - x and y are numbers ranging from 1 to 5000; preferably, x ranges from 10 to 2000 and especially from 100 to 1000; preferably, y ranges from 1 to 100;
   - R₁ and R₂, which may be identical or different, preferably identical, are linear or branched, saturated or unsaturated alkyl radicals, comprising 6 to 30 carbon atoms, preferably 8 to 24 carbon atoms and especially 12 to 20 carbon atoms;
   - A denotes a linear or branched alkylene radical containing from 2 to 8 carbon atoms,

Preferably, A comprises 3 to 6 carbon atoms, especially 4 carbon atoms; preferably, A is branched. Mention may be made especially of the following divalent radicals: -CH₂CH₂CH₂ and -CH₂CH(CH₃)CH₂-.

Preferably, R₁ and R₂, which may be identical or different, are saturated linear alkyl radicals comprising 6 to 30 carbon atoms, preferably 8 to 24 carbon atoms and especially 12 to 20 carbon atoms; mention may be made in particular of dodecyl, tetradecyl, pentadecyl, hexadecyl, heptadecyl, octadecyl, nonadecyl and eicosyl radicals; and preferentially, R₁ and R₂, which may be identical or different, are chosen from hexadecyl (cetyl) and octadecyl (stearyl) radicals.

Preferentially, the silicone is of formula (K) with:
- x ranging from 10 to 2000 and especially from 100 to 1000;
- y ranging from 1 to 100;
- A comprising 3 to 6 carbon atoms and especially 4 carbon atoms; preferably, A is branched; and more particularly A is chosen from the following divalent radicals: CH₂CH₂CH₂ and -CH₂CH(CH₃)CH₂-; and
- R₁ and R₂, which may be identical or different, being linear, saturated alkyl radicals comprising 6 to 30 carbon atoms, preferably 8 to 24 carbon atoms and especially 12 to 20 carbon atoms; chosen in particular from dodecyl, tetradecyl, pentadecyl, hexadecyl, heptadecyl, octadecyl, nonadecyl and eicosyl radicals; preferentially, R₁ and R₂, which may be identical or different, being chosen from hexadecyl (cetyl) and octadecyl (stearyl) radicals.

A preferred silicone of formula (K) is bis-cetearylamodimethicone (INCI name).

Mention may be made especially of the silicone sold under the name Silsoft^{™} AX by Momentive.

Preferably, the amino silicones according to the invention are chosen from the amino silicones of formula (F). A preferred silicone of formula (F) is amodimethicone (INCI name) sold under the tradename XIAMETER^{®} MEM-8299 Cationic Emulsion by Dow Corning.

Implementation of the present disclosure is provided by way of the following examples. The examples serve to illustrate the technology without being limiting in nature.

### Example 1

### (Booster Composition)

| INCI US | **#1** |
|---|---|
| TAURINE | 3 |
| CITRIC ACID | 3 |
| POTASSIUM HYDROXIDE | 1.3 |
| PRESERVATIVE (PHENOXYETHANOL) | 0.8 |
| WATER | Q.S. 100 |

### Example 2

### (Conditioner)

| | INCI US | **#3** |
|---|---|---|
| Cationic Surfactant(s) | BEHENTRIMONIUM CHLORIDE, CETRIMONIUM CHLORIDE, AND/OR POLYSORBATE 20 | 3.5 |
| Fatty Compound | STEARYL ALCOHOL AND/OR 2-OLEAMIDO-1,3-OCTADECANEDIOL | 1.4 |
| Water-Soluble Solvent | PROPYLENE GLYCOL AND/OR ISOPROPYL ALCOHOL | 1.1 |
| Silicone | AMODIMETHICONE | 0.9 |
| Nonionic Surfactant(s) | POLYSORBATE 20 AND/OR TRIDECETH-6 | 0.4 |
| Thickener(s) | OPTIONAL COMPONENT | 0-2 |
| Preservative(s) | OPTIONAL COMPONENT | 0-2 |
| Fragrance(s) | OPTIONAL COMPONENT | 0-2 |
| Water | WATER | Q.S. 100 |

### Example 3

### (Conditioner)

| | INCI US | **#4** |
|---|---|---|
| Cationic Surfactant(s) | BEHENTRIMONIUM CHLORIDE | 1.6 |
| Fatty Compound(s) | UNDECANE, TRIDECANE, JOJOBA SEED OIL AND/OR AVOCADO OIL | 14 |
| Silicone | AMODIMETHICONE | 1.1 |
| Nonionic Surfactant(s) | TRIDECETH-5, TRIDECETH-10, PEG-8 ISOSTEARATE | 2.5 |
| Water-Soluble Solvent(s) | GLYCERIN, ALCOHOL, AND/OR ISOPROPYL ALCOHOL | 19 |
| Preservative(s) | OPTIONAL COMPONENT | 0-2 |
| Fragrances(s) | OPTIONAL COMPONENT | 0-2 |
| | WATER | Q.S. 100 |

### Example 4

### (In Vivo Testing)

The booster composition of Example 1 was compared with a commercial benchmark booster composition that is marketed as providing nourishment, shine, body, density, and repairing benefits to hair. Each of the booster compositions were separately mixed with the conditioner of Example 2 and the conditioner of Example 3 at a ratio of 1:2 (booster composition : conditioner); 3 grams of booster composition was added to 6 grams of conditioner and mixed.

In a first comparative test, the hair of six volunteers was washed with a commercially available shampoo. While the washed hair was still damp, half of the heads of six volunteers was treated with the conditioner of Example 2 mixed with the booster composition of Example 1. The other half of the heads of these six volunteers was treated with conditioner of Example 2 mixed with the commercially available booster composition.

Separately, in a second comparative test, the hair of six volunteers was washed with a commercially available shampoo. While the washed hair was still damp, half of the heads of six other volunteers was treated with the conditioner of Example 3 mixed with the booster composition of Example 1. The other half of the heads of these six volunteers was treated with the conditioner of Example 3 mixed with the commercially available booster composition. The mixed conditioners were allowed to remain on the hair for 5 minutes before being rinsed from the hair. The hair was then dried by experts that also evaluated the products and properties of the hair. The experts evaluated the product and the hair during application of the mixed conditioners, the rinsing of the mixed conditioners, and the drying process. The results appear in the tables below. In the tables, the number has the following definitions:
"+2" Indicates a significant improvement with the Booster of Example 1
"+1" Indicates a moderate improvement with Booster of Example 1

| **Conditioner of Example 2 with Booster Composition of Example 1 v. Commercial Benchmark Booster Composition** | |
|---|---|
| Usage Qualities | |
| Suppleness during application | +2 |
| Smoothness to the touch | +1 |
| Suppleness when rinsing | +1 |

| Wet Hair | |
|---|---|
| Detangling | +2 |
| Coating | +2 |

| **Conditioner of Example 3 with Booster Composition of Example 1 v. Commercial Benchmark Booster Composition** | |
|---|---|
| Dry Hair | |
| Visual Smoothness | +1 |

As shown in the tables, overall, the booster composition of Example 1 outperformed the commercial benchmark. The booster composition of Example 1 provided moderately and significantly better results with respect to a variety of important characteristics.

The foregoing description illustrates and describes the disclosure. Additionally, the disclosure shows and describes only the preferred embodiments but, as mentioned above, it is to be understood that it is capable to use in various other combinations, modifications, and environments and is capable of changes or modifications within the scope of the invention concepts as expressed herein, commensurate with the above teachings and/or the skill or knowledge of the relevant art. The embodiments described herein above are further intended to explain best modes known by applicant and to enable others skilled in the art to utilize the disclosure in such, or other, embodiments and with the various modifications required by the particular applications or uses thereof. Accordingly, the description is not intended to limit the invention to the form disclosed herein. Also, it is intended to the appended claims be construed to include alternative embodiments.

As used herein, the terms "comprising," "having," and "including" (or "comprise," "have," and "include") are used in their open, non-limiting sense.

The terms "a," "an," and "the" are understood to encompass the plural as well as the singular.

Thus, the term "a mixture thereof" also relates to "mixtures thereof." Throughout the disclosure, the term "a mixture thereof" is used, following a list of elements as shown in the following example where letters A-F represent the elements: "one or more elements selected from the group consisting of A, B, C, D, E, F, and a mixture thereof." The term, "a mixture thereof" does not require that the mixture include all of A, B, C, D, E, and F (although all of A, B, C, D, E, and F may be included). Rather, it indicates that a mixture of any two or more of A, B, C, D, E, and F can be included. In other words, it is equivalent to the phrase "one or more elements selected from the group consisting of A, B, C, D, E, F, and a mixture of any two or more of A, B, C, D, E, and F."

Likewise, the term "a salt thereof" also relates to "salts thereof." Thus, where the disclosure refers to "an element selected from the group consisting of A, B, C, D, E, F, a salt thereof, and a mixture thereof," it indicates that that one or more of A, B, C, D, and F may be included, one or more of a salt of A, a salt of B, a salt of C, a salt of D, a salt of E, and a salt of F may be include, or a mixture of any two of A, B, C, D, E, F, a salt of A, a salt of B, a salt of C, a salt of D, a salt of E, and a salt of F may be included.

The salts, for example, the salts of the amino acids, the amino sulfonic acids, and the non-polymeric mono, di, and/or tricarboxylic acids, which are referred to throughout the disclosure may include salts having a counter-ion such as an alkali metal, alkaline earth metal, or ammonium counterion. This list of counterions, however, is non-limiting.

The expression "one or more" means "at least one" and thus includes individual components as well as mixtures/combinations.

Other than in the operating examples, or where otherwise indicated, all numbers expressing quantities of ingredients and/or reaction conditions are to be understood as being modified in all instances by the term "about," meaning within +/-5% of the indicated number.

All percentages, parts and ratios herein are based upon the total weight of the compositions of the present invention, unless otherwise indicated.

"Keratinous substrates" as used herein, includes, but is not limited to keratin fibers such as hair and/or scalp on the human head.

"Conditioning" as used herein means imparting to one or more hair fibers at least one property chosen from combability, moisture-retentivity, luster, shine, and softness. The state of conditioning can be evaluated by any means known in the art, such as, for example, measuring, and comparing, the ease of combability of the treated hair and of the untreated hair in terms of combing work (gm-in), and consumer perception.

The term "treat" (and its grammatical variations) as used herein refers to the application of the compositions of the present disclosure onto the surface of keratinous substrates such as hair. The term 'treat" (and its grammatical variations) as used herein also refers to contacting keratinous substrates such as hair with the compositions of the present disclosure.

A "rinse-off" product refers to a composition such as a hair-treatment composition that is rinsed and/or washed with water either after or during the application of the composition onto the keratinous substrate, and before drying and/or styling said keratinous substrate. At least a portion, and typically most, of the composition is removed from the keratinous substrate during the rinsing and/or washing.

The term "stable" as used herein means that the composition does not exhibit phase separation and/or crystallization for a period of time, for example, for at least 1 day (24 hours), one week, one month, or one year.

"Volatile", as used herein, means having a flash point of less than about 100°C.

"Non-volatile", as used herein, means having a flash point of greater than about 100°C.

As used herein, all ranges provided are meant to include every specific range within, and combination of sub ranges between, the given ranges. Thus, a range from 1-5, includes specifically 1, 2, 3, 4 and 5, as well as sub ranges such as 2-5, 3-5, 2-3, 2-4, 1-4, etc.

The term "substantially free" or "essentially free" as used herein means that there is less than about 5% by weight of a specific material added to a composition, based on the total weight of the compositions. Nonetheless, the compositions may include less than about 3 wt.%, less than about 2 wt.%, less than about 1 wt.%, less than about 0.5 wt.%, less than about 0.1 wt.%, or none of the specified material.

All ranges and values disclosed herein are inclusive and combinable. For examples, any value or point described herein that falls within a range described herein can serve as a minimum or maximum value to derive a sub-range, etc.

All publications and patent applications cited in this specification are herein incorporated by reference, and for any and all purposes, as if each individual publication or patent application were specifically and individually indicated to be incorporated by reference. In the event of an inconsistency between the present disclosure and any publications or patent application incorporated herein by reference, the present disclosure controls.

### NUMBERED EMBODIMENTS

[1] A kit comprising:
   (A) one or more booster compositions, the one or more booster compositions comprising:
      (a) at least 0.5 wt.% of at least one amino acid or amino sulfonic acid, and/or a salt thereof;
      (b) at least 0.5 wt.% of at least one non-polymeric mono, di, or tricarboxylic acid, and/or a salt thereof; and
      (c) water; and
   (B) one or more hair-treatment compositions;
      wherein the booster composition(s) and the hair-treatment composition(s) are separately contained.
[2] A kit of embodiment 1, wherein the at least one amino acid or amino sulfonic acid, and/or a salt thereof, is selected from the group consisting of aspartic acid, cysteine, glycine, lysine, methionine, proline, tyrosine, phenylalanine, carnitine, taurine, betaine, a salt thereof, and a mixture thereof.
[3] A kit of embodiment 1 or embodiment 2, wherein the at least one amino acid is taurine, and/or a salt thereof.
[4] A kit of any one of the above embodiments, wherein the booster(s) comprise at least 0.5 wt.% to about 40 wt.% of at least one amino acid or amino sulfonic acid, and/or a salt thereof.
[5] A kit of any one of the above embodiments, wherein the at least one non-polymeric mono, di, or tricarboxylic acid is a tricarboxylic acid, and/or a salt thereof.
[6] A kit of embodiment 5, wherein the tricarboxylic acid, and/or salt thereof, is selected from the group consisting of citric acid, isocitric acid, aconitic acid, propane-1,2,3-tricarboxylic acid, trimesic acid, a salt thereof, and a mixture thereof.
[7] A hair-treatment composition of embodiment 6, wherein the tricarboxylic acid, and/or salt thereof, is citric acid, and/or a salt thereof.
[8] A kit of any one of the above embodiments, wherein a booster composition and a hair-treatment composition are mixed prior to application to hair.
[9] A kit of any one of embodiments 1-7, wherein a booster composition and a hair-treatment composition are separately applied to hair in a sequence.
[10] A kit of any one of the above embodiments, wherein a booster composition comprises one or more surfactants.
[11] A kit of embodiment 10, wherein the one or more surfactants in the booster composition is selected from the group consisting of nonionic surfactants, cationic surfactants, and a mixture thereof.
[12] A kit of any one of the above embodiments, wherein the one or more hair-treatment compositions comprise one or more surfactants.
[13] A kit of any one of the above embodiments, wherein at least one of the one or more hair-treatment compositions is a shampoo.
[14] A kit of embodiment 7, wherein the shampoo comprises one or more anionic surfactants, amphoteric surfactants, cationic surfactants, or a mixture thereof.
[15] A kit of any one of embodiments 1-12, wherein at least one of the one or more hair-treatment compositions is a conditioner.
[16] A kit of embodiment 15, wherein the conditioner comprises one or more cationic surfactants.
[17] A kit of any one of the above embodiments comprising two or more hair-treatment compositions.
[18] A kit comprising:
   (A) a booster composition, the booster composition comprising:
      (a) at least 0.5 to about 25 wt.% at least one amino acid or amino sulfonic acid, and/or a salt thereof; and
      (b) at least 0.5 wt.% to about 25 wt.% of at least one non-polymeric mono, di, or tricarboxylic acid, and/or a salt thereof;
      (c) optionally, about 0.1 to about 10 wt.% of one or more surfactants selected from the group consisting of nonionic surfactants, cationic surfactants, and a mixture thereof; and
      (d) water;
   (B) a hair-treatment composition that is a shampoo, the shampoo comprising:
      (a) about 1 to about 25 wt.% of one or more surfactants selected from the group consisting of anionic surfactants, amphoteric surfactants, cationic surfactants, or a mixture thereof; and
      (b) water;
   (C) a hair-treatment composition that is a conditioner, the conditioner comprising;
      (a) about 1 to about 15 wt.% of one or more cationic surfactants; and
      (b) water;
      wherein the booster composition, the shampoo), and the conditioner are separately contained.
[19] A kit of embodiment 18, wherein the booster composition is mixed with the shampoo, the conditioner, or both the shampoo and the conditioner, prior to application to hair.
[20] A kit of embodiment 18, wherein the booster composition, the shampoo, and the conditioner are separately applied to the hair in a sequence.
[21] A kit of any one of the above embodiments, wherein one or more of the hair-treatment compositions comprises:
   - one or more fatty compounds.
[22] A kit of embodiment 21, wherein the one or more fatty compounds are selected from the group consisting of oils, mineral oil, alkanes, fatty alcohols, fatty acids, fatty alcohol derivatives, fatty acid derivatives (such as alkoxylated fatty acids or polyethylene glycol esters of fatty acids or propylene glycol esters of fatty acids or butylene glycol esters of fatty acids or esters of neopentyl glycol and fatty acids or polyglycerol/glycerol esters of fatty acids or glycol diesters or diesters of ethylene glycol and fatty acids or esters of fatty acids and fatty alcohols, esters of short chain alcohols and fatty acids), esters of fatty alcohols, hydroxy-substituted fatty acids, waxes, triglyceride compounds, lanolin, ceramide, and a mixture thereof .
[23] A kit of embodiment 21 or embodiment 22, wherein the one or more hair-treatment compositions comprises about 1 to about 25 wt.% of the one or more fatty compounds.
[24] A kit of any one of the above embodiments, w**h**erein one or more of the hair-treatment compositions comprises:
   - one or more silicones.
[25] A kit of embodiment 24, wherein the one or more silicones is selected from the group consisting of polyorganosiloxanes, polyalkylsiloxanes, polyarylsiloxanes, polyalkarylsiloxanes, polyestersiloxanes, and mixtures thereof.
[26] A kit of embodiment 24 or embodiment 25, wherein the one or more hair-treatment compositions comprises 0.1 to about 20 wt.% of the one or more silicones.
[27] A kit of any one of the above embodiments, wherein one or more of the hair-treatment compositions comprises:
   - one or more cationic polymers.
[28] A kit of embodiment 27, wherein the one or more cationic polymers are selected from the group consisting of poly(methacryloyloxyethyl trimethylammonium chloride), polyquaternium-37, quaternized cellulose derivatives, polyquaternium-4, polyquaternium-10, cationic alkyl polyglycosides, cationized honey, cationic guar derivatives, polymeric dimethyl diallyl ammonium salts and copolymers thereof with esters and amides of acrylic acid and methacrylic acid, copolymers of vinyl pyrrolidone with quaternized derivatives of dialkylaminoalkyl acrylate and methacrylate, vinyl pyrrolidone-vinyl imidazolium methochloride copolymers, quaternized polyvinyl alcohol, polyquaternium-2, polyquaternium-7, polyquaternium-17, polyquaternium-18, polyquaternium-24, polyquaternium-27, and a mixture thereof.
[29] A kit of embodiment 27 or embodiment 28, wherein the one or more hair-treatment compositions comprises about 0.1 to about 10 wt.% of the one or more cationic polymers.
[30] A kit of any one of the above embodiments, wherein one or more of the hair-treatment compositions comprises one or more cationic surfactants.
[31] A kit of embodiment 30, wherein the one or more cationic surfactants are selected from the group consisting of cetrimonium chloride, stearimonium chloride, behentrimonium chloride, behentrimonium methosulfate, behenamidopropyltrimonium methosulfate, stearamidopropyltrimonium chloride, arachidtrimonium chloride, distearyldimonium chloride, dicetyldimonium chloride, tricetylmonium chloride, oleamidopropyl dimethylamine, linoleamidopropyl dimethylamine, isostearamidopropyl dimethylamine, oleyl hydroxyethyl imidazoline, stearamidopropyldimethylamine, behenamidopropyldimethylamine, behenamidopropyldiethylamine, behenamidoethyldiethyl-amine, behenamidoethyldimethylamine, arachidamidopropyldimethylamine, arachidamido-propyidiethylamine, arachidamidoethyidiethylamine, arachidamidoethyidimethylamine, and a mixture thereof.
[32] A kit of embodiment 30 or embodiment 31, wherein the one or more hair-treatment compositions comprises about 1 to about 15 wt.% of the one or more cationic surfactants.
[33] A kit of any one of the above embodiments, wherein one or more of the hair-treatment compositions comprises one or more anionic surfactants.
[34] A kit of embodiment 33, wherein the one or more anionic surfactants are selected from the group consisting of alkyl sulfates, alkyl ether sulfates, acyl isethionates, acyl glycinates, acyl taurates, acyl amino acids, acyl sarcosinates, sulfosuccinates, sulfonates, and a mixture thereof, wherein the alkyl and acyl groups of all these compounds comprise from 6 to 24 carbon atoms.
[35] A kit of embodiment 33 or embodiment 34, wherein the one or more hair-treatment compositions comprises about 5 to about 25 wt.% of one or more anionic surfactants.
[36] A kit of any one of the above embodiments, wherein one or more of the hair-treatment compositions comprises one or more amphoteric surfactants.
[37] A kit of embodiment 36, wherein the one or more amphoteric surfactants are selected from the group consisting of betaines, sultaines, amphoacetates, amphoproprionates, and a mixture thereof.
[38] A method for treating hair with a kit of any one of the above embodiments comprising applying to the hair one or more hair-treatment compositions and one or more booster compositions.
[39] A method of embodiment 38 comprising mixing one or more booster compositions with one or more hair-treatment compositions prior to application to hair.
[40] The method of embodiment 39, wherein the ratio of booster composition to hair-treatment composition is about 5:1 to about 1:5, about 1:1 to about 1:5, or about 1:1 to about 1:2.
[41] A method of embodiment 38 comprising applying a booster composition to the hair before, after, or simultaneously with application of one or more hair-treatment compositions.
[42] A method for treating hair with a kit of embodiment 18 comprising:
   (i) cleansing the hair with a shampoo;
   (ii) conditioning the hair with a conditioner; and
   (iii) applying a booster composition to the hair.
[43] A method of embodiment 41, wherein:
   - the booster composition is mixed with the shampoo prior to cleansing the hair,
   - the booster composition is mixed with the conditioner prior to conditioning the hair, or
   - the booster composition is mixed with the shampoo prior to cleansing the hair and also mixed with the conditioner prior to conditioning the hair.
[44] A method of embodiment 43, wherein the ratio of booster to shampoo and/or to conditioner is about 5:1 to about 1:5, about 1:1 to about 1:5, or about 1:1 to about 1:2.
[45] A method for treating hair comprising:
   A. mixing a booster composition with a shampoo and/or a conditioner prior to application of the shampoo and/or conditioner to the hair, the booster composition comprising:
      (a) at least 0.5 wt.% of at least one amino acid or amino sulfonic acid, and/or a salt thereof; and
      (b) at least 0.5 wt.% of at least one non-polymeric mono, di, or tricarboxylic acid, and/or a salt thereof; and
      (c) water; and
   B. cleansing the hair with the shampoo and/or conditioning the hair with the conditioner,
      the shampoo comprising:
      (a) about 1 to about 25 wt.% of one or more surfactants selected from the group consisting of anionic surfactants, amphoteric surfactants, cationic surfactants, or a mixture thereof; and
      (b) water; and
      the conditioner comprising:
      (a) about 1 to about 15 wt.% of one or more cationic surfactants; and
      (b) water.
[46] A method of embodiment 45, wherein the mixed shampoo and/or the mixed conditioner is allowed to remain on the hair for about 30 second to about 30 minutes before rinsing from the hair.
[47] A method of embodiment 45, wherein the ratio of booster to shampoo and/or to conditioner is about 5:1 to about 1:5, about 1:1 to about 1:5, or about 1:1 to about 1:2.

## Claims

1. A process for strengthening, protecting, and/or repairing hair, comprising:
A. applying to the hair a restructuring composition comprising:
(a) from about 0.5% to about 15% of one or more non-polymeric carboxylic acids chosen from monocarboxylic acids, dicarboxylic acids, tricarboxylic acids, salts thereof, and combinations thereof;
(b) from about 0.5% to about 5% of one or more amino acids, salts thereof, and combinations thereof;
(c) one or more surfactants; and
(d) from about 50% to about 99% water; and
B. rinsing the hair,
wherein all amounts are by weight, relative to the total weight of the restructuring composition.

2. The process according to claim 1, wherein the (a) one or more non-polymeric carboxylic acids and salts thereof is chosen from formic acid, acetic acid, propionic acid, butyric acid, valeric acid, caproic acid, entanthic acid, caprylic acid, pelargonic acid, capric acid, undecylic acid, lauric acid, tridecylic acid, myristic acid, pentadecylic acid, palmitic acid, margaric acid, stearic acid, nonadecylic acid, arachidic acid, lactic acid, oxalic acid, malonic acid, malic acid, glutaric acid, citraconic acid, succinic acid, adipic acid, tartaric acid, fumaric acid, maleic acid, sebacic acid, azelaic acid, dodecanedioic acid, phthalic acid, isophthalic acid, terephthalic acid, 2,6-naphthalene dicarboxylic acid, citric acid, isocitric acid, aconitric acid, propane-1,2,3-tricarboxylic acid, benzene-1,3,5-tricarboxylic acid, and salts thereof.

3. The process according to claim 1 or claim 2, wherein the (a) one or more non-polymeric carboxylic acids comprises at least one tricarboxylic acid or a salt thereof, and preferably comprises at least citric acid or a salt thereof.

4. The process according to any one of claims 1-3, wherein the total amount of the (a) one or more non-polymeric carboxylic acids and salts thereof ranges from about 0.5% to about 10%, preferably from about 0.5% to about 5%, more preferably from about 2% to about 5% by weight, relative to the total weight of the restructuring composition.

5. The process according to any one of claims 1-4, wherein the (b) one or more amino acids and salts thereof is chosen from hydrophobic proteinogenic amino acids and salts thereof, and preferably comprises one or more of alanine, isoleucine, leucine, phenylalanine, valine, proline, glycine, or a salt thereof.

6. The process according to any one of claims 1-5, wherein the total amount of the (b) one or more amino acids and salts thereof is about 0.5% by weight, relative to the total weight of the restructuring composition.

7. The process according to any one of claims 1-6, wherein the (c) one or more surfactants is chosen from anionic surfactants, amphoteric surfactants, cationic surfactants, and combinations thereof, and preferably comprises one or more anionic surfactants, amphoteric surfactants, or a combination thereof.

8. The process according to any one of claims 1-7, wherein the total amount of water ranges from about 70% to about 90% by weight, based on the total weight of the restructuring composition.

9. The process according to any one of claims 1-8, wherein the restructuring composition has a pH less than 7, preferably ranging from 2.5 to 6.5, and more preferably ranging from 3 to 5.5.

10. The process according to any one of claims 1-9, wherein the restructuring composition comprises:
(a) from about 0.5% to about 5%, preferably from about 2% to about 5%, of one or more non-polymeric carboxylic acids chosen from tricarboxylic acids, salts thereof, and combinations thereof;
(b) from about 0.5% to about 5% of one or more amino acids chosen from hydrophobic proteinogenic amino acids, salts thereof, and combinations thereof;
(c) one or more surfactants chosen from anionic surfactants, amphoteric surfactants, and combinations thereof; and
(d) from about 70% to about 90% water.

11. The process according to any one of claims 1-10, wherein the restructuring composition comprises:
(a) from about 0.5% to about 5%, preferably from about 2% to about 5%, of one or more non-polymeric carboxylic acids chosen from citric acid, a salt thereof, or a combination thereof, and optionally at least one additional non-polymeric carboxylic acid or salt thereof;
(b) from about 0.5% to about 5% of one or more hydrophobic proteinogenic amino acids and salts thereof chosen from alanine, isoleucine, leucine, phenylalanine, valine, proline, glycine, salts thereof, and combinations thereof;
(c) one or more surfactants chosen from anionic surfactants, amphoteric surfactants, and combinations thereof; and
(d) from about 70% to about 90% water.

12. The process according to any one of claims 1-11, wherein the restructuring composition comprises at least one non-polymeric tricarboxylic acid or a salt thereof, and further comprises one or more additional non-polymeric carboxylic acids chosen from monocarboxylic acids, dicarboxylic acids, salts thereof, and combinations thereof.

13. The process according to any one of claims 1-12, wherein the restructuring composition comprises at least one amino acid chosen from hydrophobic proteinogenic amino acids or a salt thereof, and further comprises one or more additional amino acids chosen from amino acids other than hydrophobic proteinogenic amino acids, salts thereof, and combinations thereof.

14. The process according to any one of claims 1-13, wherein the restructuring composition is a hair treatment composition that is on the hair for a period of time ranging from about 5 seconds to about 30 minutes, preferably from about 5 second to about 20 minutes, more preferably from about 5 seconds to about 10 minutes, more preferably still from about 5 seconds to about 5 minutes, most preferably from about 5 seconds to about 1 minute before the hair is rinsed.

15. The process according to any one of claims 1-14, wherein the restructuring composition is a hair treatment composition that is applied to the hair for about 5 seconds to about 1 minute and rinsed, before a conditioner composition is applied to the hair.
